# EUROPEAN PATENT SPECIFICATION

(11) **EP 1 944 038 B1**
(45) Date of publication and mention of the grant of the patent: **27.11.2013**
(21) Application number: 06791279.0
(22) Date of filing: 18.09.2006
(51) Int. Cl.: A61K 39/12, A61K 39/295, C07K 14/18

(54) **DENGUE VIRUS CAPSID PROTEIN WHICH INDUCES A PROTECTIVE RESPONSE AND PHARMACEUTICAL COMPOSITION**
DENGUE-VIRUS-CAPSID-PROTEIN ZUR INDUKTION EINER SCHÜTZENDEN REAKTION UND PHARMAZEUTISCHE ZUSAMMENSETZUNG
PROTEINE DE LA CAPSIDE DU VIRUS DE LA DENGUE INDUISANT UNE REPONSE PROTECTRICE ET COMPOSITION PHARMACEUTIQUE ASSOCIEE

(30) Priority: 16.09.2005 CU 1682005
(43) Date of publication of application: 16.07.2008
(60) Divisional application: 11177592.0
(73) Proprietor: CENTRO DE INGENIERIA GENETICA Y BIOTECNOLOGIA, Ciudad de la Habana 10600 (CU)
(72) Inventor: LAZO VAZQUEZ, Laura, Ciudad De La Habana 10700 (CU); HERMIDA CRUZ, Lisset, Ciudad De La Habana 11000 (CU); LOPEZ ABARRATEGUI, Carlos, Ciudad De La Habana 11800 (CU); SIERRA VAZQUEZ, Beatriz de la Caridad, Ciudad De La Habana 17100 (CU); VAZQUEZ RAMUNDO, Susana, Ciudad De La Habana 17100 (CU); VALDEZ PRADO, Iris, Ciudad De La Habana 10700 (CU); GUILLEN NIETO, Gerardo, Enrique, Ciudad De La Habana 10400 (CU); GUZMAN TIRADO, María, Guadalupe, Ciudad De La Habana 11300 (CU); ZULUETA MORALES, Aída, Ciudad De La Habana 17200 (CU)
(74) Representative: Jansen, Cornelis Marinus
(86) International application number: PCT/CU2006/000008
(87) International publication number: WO 2007/031034

(56) References cited:
- EP-A- 1 454 988
- EP-A2- 1 418 180
- WO-A-93/06214
- WO-A2-99/18216
- LAZO L ET AL: "A recombinant capsid protein from Dengue-2 induces protection in mice against homologous virus" VACCINE 22 JAN 2007 UNITED KINGDOM, vol. 25, no. 6, 22 January 2007 (2007-01-22), pages 1064-1070, XP002418880 ISSN: 0264-410X

## Description

The scope of the present invention is defined by the claims and any information that does not fall within the claims is provided for information only.

The present invention is related to the field of biotechnology and the pharmaceutical industry, in particular to obtaining proteins capable of inducing an immune response against infection with Dengue virus, quoted from now on as DEN, avoiding the antibody-dependent enhancement phenomenon described in persons re-infected with this virus.

Dengue fever (DF) and dengue hemorrhagic fever (DHF) are becoming increasingly important as health problems, affecting many countries of the tropical and subtropical zones of our planet. Dengue virus has been recognized in more than 100 countries and an estimated 2 500 million people are living in risk areas. Between 50 and 100 million cases from DF and 250 000 to 500 000 of DHF are reported each year. (Guzman M.G. and Kouri G. 2002. Dengue: an update. Lancet Infect. Dis. 2: 33-42).

The causal agent of this disease is the Dengue virus, belonging to the family Flaviviridae, genus Flavivirus, which is transmitted by the mosquito *Aedes aegypti* (Leyssen P., De Clerco E., Neyts J. 2000. Perspectives for the treatment of infections with Flaviviridae. Clin. Microbiol. Rev. 13: 67-82).

Until now four serotypes have been reported that can circulate in the same region. Dengue virus is an enveloped positive RNA virus, whose genome contains a single reading frame that is translated in a polyprotein that is processed in three structural proteins and seven non-structural proteins. (Russell P.K., Brandt W.E., Dalrymple J.M. 1980. Chemical and antigenic structure of flaviviruses. The togaviruses: biology, structure, replication. Schelesinger R.W. (ed.). 503-529).

Multiple epidemiological studies have been made to determine the risk factors that are involved with the most severe form of Dengue disease, which is characterized by high fever, fluid extravasation, bleeding and ultimately Dengue shock. (Gubler D.J. 1998. Dengue and Dengue Hemorrhagic Fever. Clin. Microbiol. Rev. 11: 480-496). One of the most important risk factors is the secondary infection by a heterologous serotype, which implies that there is no.cross-protection between infections of different serotypes. (Kouri G., Guzman M.G., Bravo J., Trina C. 1989. Dengue hemorrhagic fever/dengue shock syndrome: lessons from the Cuban epidemic. WHO Bulletin OMS. 67: 375-380).

Several hypotheses exist to explain this phenomenon, among which is the antibody depend enhancement. (Halstead S.B., Scanlon J.E., Umpaivit P., Udomsakdi S. 1969. Dengue and Chikungunya virus infection in man in Thailand, 1962-1964. IV. Epidemiologic studies in the Bangkok metropolitan area. Am. J. Trop. Med. Hyg. 18: 997-1021).

Early studies suggested that DEN virus replicates further in peripheral mononuclear cells in the blood of patients who had had a previous infection with this virus (Halstead S.B., O'Rourke E.J., Allison A.C. 1977. Dengue viruses and mononuclear phagocytes. II. Identity of blood and tissue leukocytes supporting in vitro infection. J. Exp. Med. 146: 218-229). Later, it was demonstrated that the residual antibodies were responsible for this effect (Morens DM, Halstead SB, Marchette NJ. 1987. Profiles of antibody-dependent enhancement of dengue virus type 2 infection. Microb Pathog. Oct;3(4):231-7).

Under conditions of specificity or concentration of antibodies in which no neutralization occurs, the antibody-virus complexes can be internalized by cells that express FCy receptors in the membranes, such as monocytes and macrophages. This mechanism, known as antibody-dependent enhancement (ADE) occurs during secondary infections. (Morens DM, Halstead SB, Marchette NJ. 1987. Profiles of antibody-dependent enhancement of dengue virus type 2 infection. Microb Pathog. Oct;3(4):231-7; Kliks S.C., Nimmannitya S., Nisalak A., Burke D.S. 1988. Evidence that maternal dengue antibodies are important in the development of dengue hemorrhagic fever in infants. Am. J. Trop. Med. Hyg. 38: 411-419).

Halstead *et al.* (Halstead S.B., Scanlon J.E., Umpaivit P., Udomsakdi S. 1969. Dengue and Chikungunya virus infection in man-in Thailand, 1962-1964. IV. Epidemiologic studies in the Bangkok metropolitan area. Am. J. Trop. Med. Hyg. 18: 997-1021.), in a 3-year study in Bangkok, Thailand, reported that the hospitalization rates for DEN infection among children reached a maximum in those between 7 and 8 months old. These rates were four to eight times greater than those observed among children of 1-3 months and twice that of children under 3 years. Kliks *et al.* (Kliks S.C., Nimmannitya S., Nisalak A., Burke D.S. 1988. Evidence that maternal dengue antibodies are important in the development of dengue hemorrhagic fever in infants. Am. J. Trop. Med. Hyg. 38: 411-419), determined the relation between the maternal neutralizing antibody titers against DEN-2 and the age of thirteen children with FHD caused by infection with the homologous virus. The results showed that the serious cases of infection with the virus occurred when the maternal antibody levels diminished considerably to subneutralizing levels. These data are consistent with the hypothesis that maternal antibodies play the dual role of initially protecting and later stimulating the development of DHF.

Despite the existence of this immunological phenomenon, nowadays the most advanced vaccine candidates worldwide are based on attenuated virus strains of the different four serotypes containing the envelope protein. These candidates are potentially capable of inducing immunostimulatory antibodies against the exposed proteins (PrM/M and Envelope proteins) and protective neutralizing antibodies against the four viral serotypes (Kanesathasan N., Sun W., Kim-Ahn G., Van Albert S., Putnak J. R., King A., Raengsakulsrach B., Christ-Schmidt H., Gilson K., Zahradnik J.M., Vaughn D.W., Innis B.L., Saluzzo J.F. y Hoke C.H. 2001. Safety and immunogenicity of attenuated dengue virus vaccines (Aventis Pasteur) in human volunteers. Vaccine. 19: 3179-3188).

If induction of high levels of neutralizing antibodies could be achieved by immunization, this could prevent viral replication despite the induction of enhancing antibodies. The problem may occur when not achieving full seroconversion of the four serotypes in the vaccinees in terms of neutralizing Abs, or as time passes neutralizing Abs decline to low levels in blood and the individuals would become then susceptible to a severe secondary infection with a viral serotype for which no protective antibodies are present. In fact, several tests in monkeys and humans have been made to define the viral amounts in the vaccine formulations. (Guirakhoo F., Arroyo J., Pugachev K.V., Miller C., Zhang Z.-X., Weltzin R., Georgakopoulos K., Catalan J., Ocran S., Soike K., Raterree M., Monath T.P. 2001. Construction, safety, and immunogenicity in nonhuman primates of a chimeric yellow fever-dengue virus tetravalent vaccine. J. Virol. 75: 7290-7304).

In some cases no balanced seroconversion was achieved against all four serotypes (Sabchareon A, Lang J, Chanthavanich P, Yoksan S, Forrat R, Attanath P, Sirivichayakul C, Pengsaa K, Pojjaroen-Anant C, Chokejindachai W, Jagsudee A, Saluzzo JF, Bhamarapravati N. 2002. Safety and immunogenicity of tetravalent live-attenuated dengue vaccines in Thai adult volunteers: role of serotype concentration, ratio, and multiple doses. Am J Trop Med Hyg. 66(3): 264-72). In addition, it has been necessary to administer up to three doses of attenuated vaccines in children for a full seroconversion in terms of neutralizing antibodies and it remains to be seen whether these will endure (Sabchareon A, Lang J, Chanthavanich P, Yoksan S, Forrat R, Attanath P, Sirivichayakul C, Pengsaa K, Pojjaroen-Anant C, Chambonneau L, Saluzzo JF, Bhamarapravati N..2004. Safety and immunogenicity of a three dose regimen of two tetravalent live-attenuated dengue vaccines in five- to twelve-year-old Thai children. Pediatr Infect Dis J.;23(2):99-1 09). This is currently one of the most questioned aspects in formulations that include the envelope protein of Dengue virus, and consequently for the vaccine candidates in development.

Other drawbacks of attenuated vaccines that are currently in phase I/II is the safety. It has been demonstrated in various studies that there are adverse effects in adults and children (fever, myalgia, petechiae and headache) following the inoculation of the first dose, has been demonstrated in several studies (Sabchareon A, Lang J, Chanthavanich P, Yoksan S, Forrat R, Attanath P, Sirivichayakul C, Pengsaa K, Pojjaroen-Anant C. Chambonneau L, Saluzzo JF, Bhamarapravati N. 2004. Safety and immunogenicity of a three dose regimen of two tetravalent live-attenuated dengue vaccines in five- to twelve-year-old Thai children. Pediatr Infect Dis J.;23(2):99-109). In general, phenomena of reversion to the potential virulence associated with live vaccines may occur.

In the search for new alternatives, different variants of vaccine candidates have been developed based on the envelope protein or fragments thereof, obtained by recombinant technology. While these strategies avoid the safety problems related to the inoculation of live virus, they represent the same disadvantage in being potentially able to sensitize the individual if the formulation is not tetravalent or does not induce an equivalent response of neutralizing antibodies against the four serotypes (Velzing J, Groen J, Drouet MT, van Amerongen G, Copra C, Osterhaus AD, Deubel V. 1999. Induction of protective immunity against Dengue virus type 2: comparison of candidate live attenuated and recombinant vaccines. Vaccine. Mar 17;17(11-12):1312-20). Moreover, those candidates which achieve a serotype-specific response require powerful adjuvants to achieve a sufficiently protective immune response, which rare-not approved for use in humans (Hermida L, Rodriguez R, Lazo L, Silva R, Zulueta A, Chinea G, Lopez C, Guzman MG, Guillen G. 2004. A dengue-2 Envelope fragment inserted within the structure of the P64k meningococcal protein carrier enables a functional immune response against the virus in mice. J Virol Methods. 2004 Jan;115(1):41-9).

The humoral response of neutralizing antibodies has been extensively studied in animals and its protective effect has been demonstrated. The cytotoxic cellular immune response as a protective mechanism in Dengue has not deeply been explored. On the contrary, there are several reports in which the correlation between the induction of a cellular response and the most severe form of the disease is demonstrated (Rothman A.L. y Ennis F.A. 1999. Immunopathogenesis of Dengue Hemorragic Fever. Virology. 257: 1-6). These studies are based on the presence of high levels of activated T-cells in those individuals that exhibit DHF (Green S, Pichyangkul S, Vaughn DW, Kalayanarooj S, Nimmannitya S, Nisalak A, Kurane I, Rothman AL, Ennis FA. 1999. Early CD69 expression on peripheral blood lymphocytes from children with dengue hemorrhagic fever. J Infect Dis. 180(5):1429-35).

T-cells epitopes have been reported mainly in nonstructural proteins (Kurane I, Zeng L, Brinton MA, Ennis FA. 1998. Definition of an epitope on NS3 recognized by human CD4+ cytotoxic T lymphocyte clones cross-reactive for dengue virus types 2, 3, and 4. Virology. 1998 Jan 20; 240(2): 169-74). T-cell epitopes have also been reported in the envelope and in the viral capsid proteins (Bukowski, J. F., I. Kurane, C.-J. Lai, M. Bray, B. Falgout, and F. A. Ennis. 1989. Dengue virus-specific cross-reactive CD8 human cytotoxic T lymphocytes. J. Virol. 63:5086-5091; Gagnon S.J., Zeng W., Kurane I., Ennis F.A. 1996. Identification of two epitopes on the dengue 4 virus capsid protein recognized by a serotype-specific and a panel of serotype-cross-reactive human CD4+ cytotoxic T-lymphocyte clones. J Virol. 70: 141-147). Nevertheless, the protective character of some of these proteins based only on the induction of a cellular immune response has not been demonstrated.

In the search for vaccine candidates that avoid the immuno-enhancement phenomenon, studies using the non-structural proteins NS1 and NS3 have been made. In the case of NS1, some level of protection in mice immunized with the recombinant protein has been reached. Similar results have been obtained using a naked DNA containing the NS1 gene, through the ADCC mechanism (Wu SF, Liao CL, Lin YL, Yeh CT, Chen LK, Huang YF, Chou HY, Huang JL, Shaio MF, Sytwu HK. 2003. Evaluation of protective efficacy and immune mechanisms of using a non-structural protein NS1 in DNA vaccine against dengue 2 virus in mice. Vaccine. Sep 8;21 (25-26):3919-29). However, there are reports of their possible roll in phenomena of autoimmunity due to the induction of antibodies that recognize human endothelial cells (Chiou-Feng Lin, Huan-Yao Lei, Ai-Li Shiau, Hsiao-Sheng Liu, Trai-Ming Yeh, Shun-Hua Chen, Ching-Chuan Liu, Shu-Chen Chiu, and Yee-Shin Lin. 2002. Endothelial Cell Apoptosis Induced by Antibodies against Dengue Virus nonstructural Protein 1 via Production of Nitric Oxide. J. Immunol. 657-664).

Additionally, there is a report of protection using a naked DNA formulation containing the gene of the NS3 protein, but, it was demonstrated that this protection was mediated by the raised antibodies since the same protection was obtained with passive immunization (Tan CH, Yap EH, Singh M, Deubel V, Chan YC. 1990. Passive protection studies in mice with monoclonal antibodies directed against the non-structural protein NS3 of dengue 1 virus. J Gen Virol. 1990 Mar:71 (Pt 3):745-9). In addition, it is worth noting that the hypothesis that the cellular response can be potentially harmful against heterologous virus inmfection is based on studies of epitopes contained in the NS3 protein (Zivny J, DeFronzo M, Jarry W, Jameson J, Cruz J, Ennis FA, Rothman AL. 1999. Partial agonist effect influences the CTL response to a heterologous dengue virusserotype. J Immunol. Sep1; 163(5):2754-60).

In the case of the capsid protein of dengue virus, no evidence of protection against challenge with lethal dengue virus is reported. In the case of related flaviviruses, a report was published where authors inoculated mice with a naked DNA formulation containing the gene of the Japanese Encephalitis (JE) capsid protein. This formulation did not induce a protective response against a challenge with lethal JE in mice, despite the demonstration of a cytotoxic response (Konishi E, Ajiro N, Nukuzuma C, Mason PW, Kurane I. 2003. Comparison of protective efficacies of plasmid DNAs encoding Japanese encephalitis virus proteins that induce neutralizing antibody or cytotoxic T lymphocytes in mice. Vaccine. Sep 8:21 (25-26):3675-83).

Protection using recombinant capsids has only been demonstrated in the case of human papilloma virus, although a possible protective role with other viruses such as Hepatitis C virus has been suggested. Nevertheless, in all cases, they are chronic infections or tumors, in which the cellular cytotoxic response is the only means of the immune system to clear viral infection (Duenas-Carrera S, Alvarez-Lajonchere L, Alvarez-Obregon JC, Herrera A, Lorenzo LJ, Pichardo D, Morales J. 2000. A truncated variant of the hepatitis C virus core induces a slow but potent immune response in mice following DNA immunization. Vaccine. Nov 22;19(7-8):992-7; Suzich JA, Ghin SJ, Palmer-Hill FJ, et al. 1995. Systemic immunization with papillomavirus L1 protein completely prevents the development of viral mucosal papillomas. Proc Natl Acad Sci USA; 92: 11553-57). These types of diseases do not correspond with the acute profile that is exhibited during infection by Dengue in humans (Vaughn D.W., Green S., Kalayanarooj S., Innis B.L., Nimmannitya S., Suntayakorn S., Endy T.P., Raengsakulrach B., Rothman A.L., Ennis F.A. y Nisalak A. 2000. Dengue viremia titer, antibody response pattern, and virus serotype correlate with disease severity. J Infect Dis.181: 2-9).

The capsid protein of Dengue virus has a molecular weight of 9 to 12 kDa (112-127 amino acids) and has a marked basic character because the 25% of its amino acids are arginine and lysine. The presence of these amino acids could favor antigenic presentations to the immune system due to the capacity of polycationic peptides to do so. (Lingnau K., Egyed A., Schellack C., Mattner F, Buschle M., Schmidt W. 2002. Poly-I -arginine synergizes with oligodeoxynucleotides containing CpG-motifs (CpG-ODN) for enhanced and prolonged immune responses and prevents the CpG-ODN-induced systemic release of pro-inflammatory cytokines. Vaccine. 20: 3498-3508). The protein is located totally within the virion structure without any exposed region (Kuhn RJ, Zhang W, Rossmann MG, Pletnev SV, Corver J, Lenches E, Jones CT, Mukhopadhyay S, Chipman PR, Strauss EG, Baker TS, Strauss JH. 2002. Structure of dengue virus: implications for flavivirus organization, maturation, and fusion. Cell. Mar 8;108(5):717-25).

Jones et al.. (Christopher T. Jones, Lixin Ma, John W. Burgner, Teresa D. Groesch, Carol B. Post, and Richard J. Kuhn. 2003. Flavivirus Capsid Is a Dimeric Alpha-Helical Protein. Journal of Virology, p7143-7149, Vol. 77, Neo. 12) purified the capsid protein of VD2 obtained by the recombinant way in *Escherichia coli (E. coli)* and demonstrated that this protein behaves like a dimer in solution in the absence of nucleic acids. Its secondary structure is mainly in the form of alpha-helices and is comprised of four of these, the longest one found at the C-terminal end. The N-terminal end does not present a defined structure and its deletion does not affect the structural integrity of the protein.

This invention describes for the first time that the capsid of DEN-2 virus, obtained by a recombinant way in *E. coli* and with only a 40% of purity, is able to induce a protective immune response against challenge with lethal DEN-2 virus in mice. It was demonstrated that the highly purified protein retained its protective capacity, which was surpassed during the immunization of mice with the particulate form of the molecule. Moreover, it was demonstrated that the protection achieved was mediated by CD8+ T-cells, a novel element considering that the reported T-cells epitopes for the capsid so far, are recognized by CD4+ T cells (Gagnon SJ, Zeng W, Kurane I, Ennis FA. 1996. Identification of two epitopes on the dengue 4 virus capsid protein recognized by a serotype-specific and a panel of serotype-cross-reactive human CD4+ cytotoxic T-lymphocyte clones. J Virol. 70(1): 141-7; Simmons CP, Dong T, Chau NV, Dung NT, Chau TN, Thao le TT, Dung NT, Hien TT, Rowland-Jones S, Farrar J. 2005. Early T-cell responses to dengue virus epitopes in Vietnamese adults with secondary dengue virus infections. J Virol. 79(9):5665-75).

Additionally, this recombinant molecule was mixed with protein PD5, which is formed by the P64k protein of *Neisseria meningitidis* and domain III of the envelope protein of the Dengue-2 virus. This fusion protein is able to generate a highly serotype-specific, protective and neutralizing immune response, with a low probability of generating the phenomenon of antibody-dependent enhancement (Hermida L, Rodriguez R, Lazo L, Silva R, Zulueta A, Chinea G, Lopez C, Guzman MG, Guillen G. 2004. A dengue-2 Envelope fragment inserted within the structure of the P64k meningococcal protein carrier enables a functional immune response against the virus in mice. J Virol Methods. 2004 Jan;115(1):41-9).

Also described is obtaining a genetic construct formed by the fusion of domain III and the capsid protein to achieve the same objective. The result was that the two formulations where the capsid and the domain III of DEN-2 are combined generated a lymphoproliferative response in mice greater than that generated by the capsid alone, and in addition a serotype specific antibody-response greater than that generated by PD5 alone. This last result demonstrates the immunoenhancing capacity of the capsid protein of dengue virus in the generation of antibodies by a heterologous antigen, a phenomenon described for other recombinant capsids from other viruses such as the hepatitis B virus (Alvarez JC, Guillén G. Formulations containing virus like particles as immunoenhancers by mucosal route. Cuban office of the Industrial property. CU 1998/183).

### Detailed description of the invention

The objective of this invention is to obtain a recombinant protein corresponding to the capsid of Dengue virus, which when inoculated in mice generates a protective response against infection with the lethal virus.

The gene encoding the capsid protein of Dengue virus was inserted into a plasmid containing the phage T5 promoter. The cells *XL-1Blue,* transformed with the recombinant plasmid, expressed high levels of the resulting protein.

This protein was semipurified to about 40% purity, and was adjuvated in aluminum hydroxide for its inoculation in Balb/c mice. Once per month after the last dose the antiviral antibody response and the lymphoproliferative response in spleen lymphocytes stimulated *in vitro* with the dengue virus, were determined. As a result it was determined that no antiviral antibodies were induced and that there was a significant lymphoproliferative response. In parallel, in non-analysed mice, the protection assay was performed. Hereto, a lethal dose corresponding to 100 LD₅₀ of Dengue virus was inoculated and the disease symptoms and death were observed during 21 days. The result was a 44% survival in mice immunized with recombinant capsid, while in the negative control group all mice died. This is the first evidence of a protective response against Dengue virus due to the inoculation with the capsid protein alone.

Then, a purification process using high-resolution chromatography was conducted to achieve a percentage of purity >95%.

Both preparations, the semipurified and purified variant, were analyzed by gel chromatography and HPLC in order to know the state of aggregation of the protein in each sample. In case of the semipurified preparation a fraction with lower retention times was detected, while in the purified sample a retention time corresponding to the dimeric form of the molecule was detected.

In order to achieve a state of aggregation for the purified variant, an *in vitro* particulation process employing low quantities of oligonucleotides was performed. As a result, particles of about 21 nm in diameter were obtained.

The dimeric and particulated variants, both with more than 95% purity, were inoculated in mice. The dimeric preparation was adjuvated with Freund's Adjuvant and aluminum hydroxide, while the particulated variant was adjuvated only with aluminum hydroxide.

Similar to what was obtained for the semipurified preparation, high levels of lymphoproliferation were detected in spleen lymphocytes stimulated *in vitro* with the dengue virus. In performing the protection assay, it was determined that with the pure dimeric variant levels of protection of 40 and 20% were achieved when adjuvated with Freund's adjuvant and aluminum, respectively, however, the pure particulated protein adjuvated with aluminum induced a higher protection percentage.

These results together with those obtained with the semipurified protein show the capacity of the capsid protein to induce a protective response in Balb/c mice, and in turn demonstrate that the particulated form of the protein is superior also allowing its future use in humans due to the use of aluminum hydroxide as adjuvant. Additionally, lack of induction of an antiviral antibody response eliminates the phenomenon of antibody-dependent enhancement as a risk factor for the occurrence of the most severe form of the disease: the dengue hemorrhagic fever.

In order to determine the possible mechanism of protection, which it is not related to the induction of antibodies as demonstrated by their absence, a study was performed on the depletion of CD8+ cells, a marker present on cytotoxic T-cells. The result was that the protection achieved with pure proteins of each variant was dependent of the presence of the cells having this marker, since their eliminating removed the induced protective effect.

Additionally, a study was performed in order to know if the combination of the particulated recombinant capsid with antigens that induce a humoral response does not affect the generation of the lymphoproliferative response and to have a mixture of immunogens able to contribute to both branches of the immune response. To this end, we inoculated the purified particulated variant of the capsid and a fusion protein containing the domain III of the envelope protein of the dengue-2 virus which is able to generate a serotype-specific immune response also minimizing the phenomenon of ADE (Hermida L, Rodriguez R, Lazo L, Silva R, Zulueta A, Chinea G, Lopez C, Guzman MG, Guillen G. 2004. A dengue-2 Envelope fragment inserted within the structure of the P64k meningococcal protein carrier enables a functional immune response against the virus in mice. J Virol Methods. 2004 Jan;115(1):41-9). The administration of three doses and the analysis of the raised antibodies demonstrated that the induction of antiviral serotype specific antibodies was enhanced as a result of the inoculation of the fusion protein. In turn, a lymphoproliferative response higher than that induced by the capsid alone and significantly higher than that induced by inoculation of only the fusion protein was detected.

Parallel to know whether it is possible to obtain the effect of the combination via genetic fusion of both antigens, a plasmid containing the domain III of the envelope protein of DEN-2 virus fused to the N-terminal of the capsid protein gene was constructed. The resulting protein, with a 40% of purity, also generated in Balb/c mice a lymphoproliferative response higher than that obtained by the capsid alone and a serotype specific antibody response higher than that induced by PD5.

### FIGURES DESCRIPTION

**Figure 1****.** Cloning strategy of the capsid protein of DEN-2 virus to generate PDC-2. DEN2 C: Fragment of the capsid protein of DEN-2.
**Figure 2****.** Analysis by SDS-PAGE at 15% of the PDC-2 semipurification process. 1. Rupture supernatant. 2 and 3. Fraction not adsorbed to *Q Sepharose FF.* 4. Fraction eluted with NaCl 1M.
**Figure 3****.** Analysis by SDS-PAGE at 15% of the PDC-2 purification process. 1. Rupture supernatant, 2. Fraction not absorbed to the gel, 3. Washed (350 mM NaCl), 4. Eluted fraction (750 mM NaCl), 5. Fraction in Tris 10 mM, EDTA 1 mM.
**Figure 4****.** Chromatographic profile in Superdex 200 of the semipurified (A) and pure (B) preparations of PDC-2.
**Figure 5****.** Electronic microscopy pictures of the pure PDC-2 preparation before (A) and after (B) the treatment with oligonucleotides.
**Figure 6****.** Cloning strategy of the capsid protein of DEN-1 virus to generate PDC-1. DEN1 C: Fragment of the capsid protein of DEN-1.

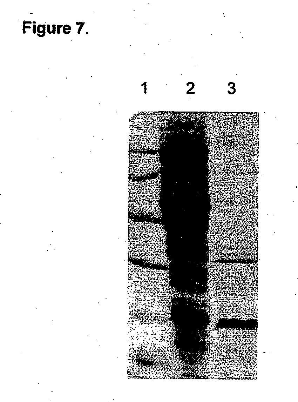

**Figure 7****.** Analysis by SDS-PAGE at 15% of the PDC-1 semipurification process. 1. Molecular weight marker. 2. Rupture supernatant. 3. Fraction not adsorbed to Q *Sepharose FF*.

### EXAMPLES

### EXAMPLE 1. Cloning and expression of PDC-2.

The nucleotide sequence that codes for amino acids 1 to 99 of the capsid protein from DEN-2 virus (Sequence No. 3), was amplified with the oligonucleotides identified in the sequence list as Sequence No. 1 and Sequence No. 2 from the DEN-2 virus strain genotype Jamaica (Deubel V., Kinney R.M., Trent D.W. Nucleotide sequence and deduced amino acid sequence of the nonstructural proteins of Dengue type 2 virus, Jamaica genotype: Comparative analysis of the full-length genome. Virology 1988.165:234-244).

The vector was created by digestion of the plasmid pQE-30 with *BamHI*/*HindIII,* which contains the phage T5 promoter and a 6-histidine tail in the N-terminal region (Sequence No. 6). Upon ligation, the potential recombinants were analyzed by restriction enzyme digestion and positive clones were sequenced to check up the junctions.

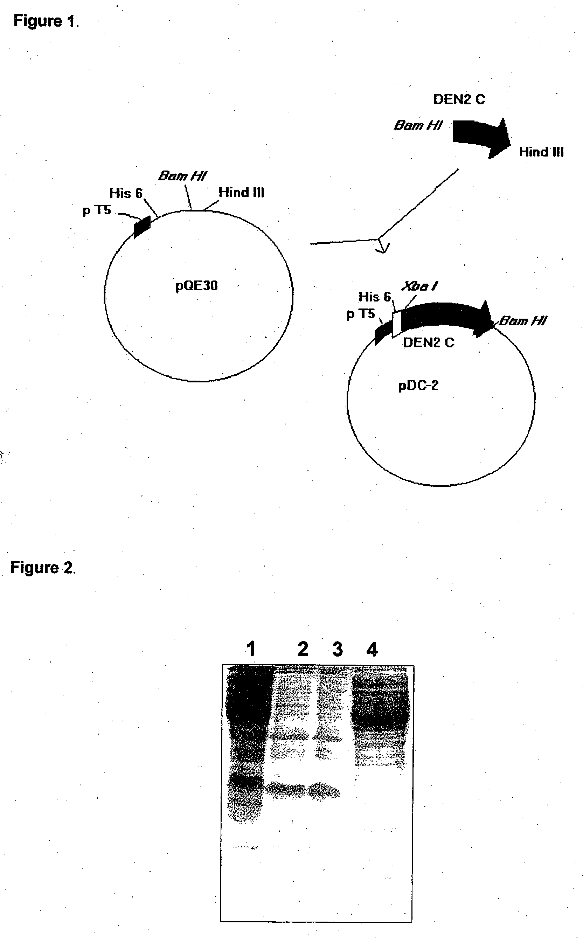

Competent cells XL-1 Blue (Hanahan D. 1983. Studies on transformation of Escherichia coli with plasmids. J. Mol. Biol. 166:557-580) were transformed with the selected clone called pDC-2 (Fig. 1 and Sequence No. 4). The transformed *E. coli* strains were cultivated in Luria Bertani medium (LB) supplemented with Ampicilline 50 µg/mL for 10 h at 37°C. Isopropyl-B-D-thiogalactopyranoside (IPTG) to a final concentration of 1mM was used for the induction of the promoter. Upon growing the colony, an SDS-PAGE of the cellular lysate was done. As a result, a 15-kDA band was obtained. The protein was recognized by an anti-DEN-2 hyperimmune ascitic fluid (HMAF). This protein was denominated PDC-2 (Sequence No. 5).

### EXAMPLE 2. Semipurification and characterization of PDC-2.

The biomass obtained from the *E. coli* strain transformed with pDC-2 and grown at 37°C was disrupted by French press. The recombinant protein was obtained equally distributed between the soluble and insoluble fractions. The soluble fraction was subjected to an anionic interchange chromatography, using a Q *Sepharose FF* column and the buffer Tris 10mM pH 8. The protein in the non-absorbed fraction was obtained with 40% purity and was used for the immunological studies (Fig. 2).

### EXAMPLE 3. Immunological evaluation of semipurified PDC-2.

Three groups of 30 Balb/c mice were used. Two of them were immunized with 10 ug of the recombinant protein by intraperitoneal route, using Freund's Adjuvant (FA) in one of the groups and aluminum hydroxide in the other. The soluble fraction resulting from the rupture of the pQE-30-transformed cells was used as negative control adjuvanted with FA; 10 animals were bled 15 days after the third dose and the antibody titers against DEN-2 were determined by ELISA. After the immunization with the recombinant protein, formulated in either adjuvant, no antibody titers were obtained.

**Table 1. Antibody titers against DEN-2 from the sera obtained upon immunization of mice with semipurified PDC-2.**

| **Mouse** | **ELISA Titers against DEN-2** | | |
|---|---|---|---|
| | **PDC-2 Freund A.** | **PDC-2 Aluminum hydroxide** | ***XL-1 Blue* Freund (Neg. Control)** |
| 1 | < 1: 100 | < 1: 100 | < 1: 100 |
| 2 | < 1: 100 | < 1: 100 | < 1: 100 |
| 3 | < 1: 100 | < 1: 100 | < 1: 100 |
| 4 | < 1: 100 | < 1: 100 | < 1: 100 |
| 5 | < 1: 100 | < 1: 100 | < 1: 100 |
| 6 | < 1: 100 | < 1: 100 | < 1: 100 |
| 7 | < 1: 100 | < 1: 100 | < 1: 100 |
| 8 | < 1: 100 | < 1: 100 | < 1: 100 |
| 9 | < 1: 100 | < 1: 100 | < 1: 100 |
| 10 | < 1: 100 | < 1: 100 | < 1: 100 |

### EXAMPLE 4. Protection assay.

For the evaluation of the protection conferred to mice against challenge with lethal homologous DEN virus by the immunization with the described variants, 10 mice were used from each of the groups immunized with the recombinant protein adsorbed in aluminum hydroxide and with the control preparation. Each animal received a dose of 100 LD₅₀ of lethal DEN-2 virus by intracranial inoculation and was observed for 21 days to obtain the percentages of lethality in terms of death by viral encephalitis. As a positive control, a group of 10 mice immunized with infective DEN-2 virus (10⁴ pfu) was used. All mice in the positive control group survived, while in the negative control group all mice were sick by day 7-11 after challenge and 100% mortality was obtained by day 21. Finally, the group immunized with the recombinant protein PDC-2 presented 44.4% protection (table 2).

**Table 2. Percentage of survival in PDC-2 immunized mice upon challenge with the homologous lethal Dengue virus.**

| Immunogen | Survival percentage |
|---|---|
| XL-1 blue | 0 |
| DEN-2 | 100 |
| PDC-2 alum. | 44,4 |

| | |
|---|---|
| * It was calculated: (# de survivors) / (total # of mice). Data of survivors were taken 21 days after challenge. | |

### EXAMPLE 5. Lymphoproliferative response

The rest of the animals from the group immunized with the capsid protein adjuvanted with aluminum hydroxide were sacrificed 30 days after the last dose. Then, their spleens were extracted and the lymphoproliferative response to DEN-2 was studied. The results in table 3 show the stimulation indexes obtained.

**Table 3. Stimulation indexes against the homologous serotype of the lymphocytes from immunized mice.**

| | **PDC-2 Aluminum hydroxide** |
|---|---|
| **DEN-2**** | 8* |
| **Control** | 1.5 |
| **Antigen***** | |
| **PHA****** | 7 |

| | |
|---|---|
| * Stimulation index: quotient average of counts / minutes of samples between counts / minutes of the ADN spontaneous synthesis control. ** Preparation of DEN-2 infected mice brain. *** Preparation of not infected mice brain. **** Phytohemaglutinina Mitogen (Positive Control). | |

### EXAMPLE 6. Purification of PDC-2

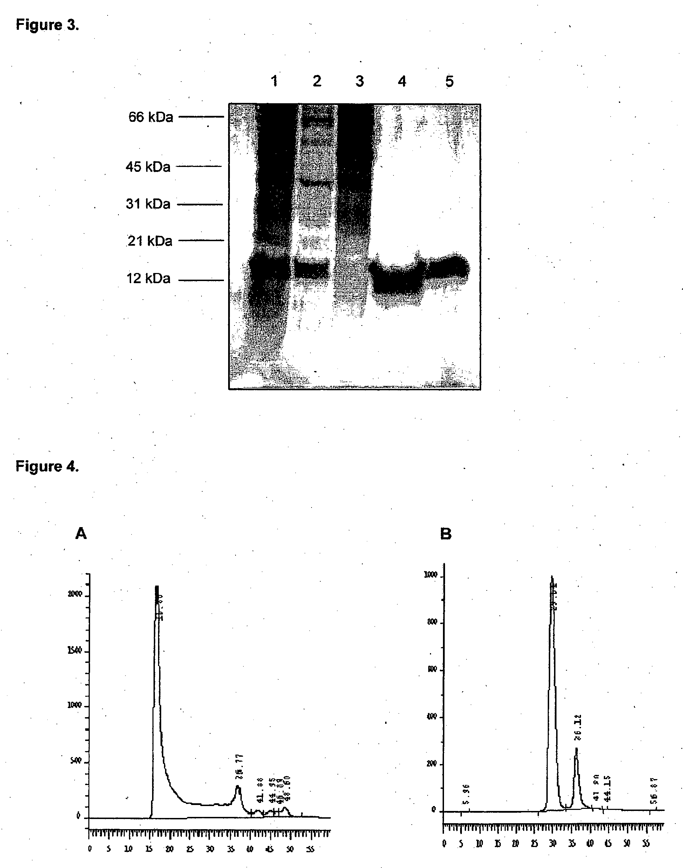

The biomass obtained from the *E. coli* strain transformed with pDC-2 and grown at 37°C was disrupted by French press. The recombinant protein was obtained equally distributed between the soluble and insoluble fractions. The soluble fraction was subjected to a cationic interchange chromatography, using an SP*-Sepharose FF* column and the buffer Tris 10mM, Tween 0.5%, urea 7M, pH 8. The column was washed with buffer diethanolamine 30mM, NaCl 350 mM, pH 10.3. The elution of the protein of interest was done with buffer diethanolamine 30mM, NaCl 750 mM, pH 10:3. Once eluted the protein, the buffer was exchanged using G-25 columns. Finally, the protein was obtained with 96% purity in buffer Tris 10 mM, EDTA 1 mM (Figure 3).

### EXAMPLE 7. Characterization of the semipurified and purified variants

With the aim of characterizing the state of aggregation of the semipurified and the purified preparations, gel filtration chromatographies were done using the TSK-5000 column (Tosoh bioscience, Japan). After applying the semipurified sample, a homogeneous and major peak was obtained, with a retention time ranging from 15 to 20 minutes, evidencing the presence of high molecular weight species (Figure 4A). Contrarily, in the sample from the highly purified fraction of the capsid protein, retention times of 30 minutes were detected, corresponding to the dimeric form of the molecule (Figure 4B).

### EXAMPLE 8. Studies of reparticulation "in vitro".

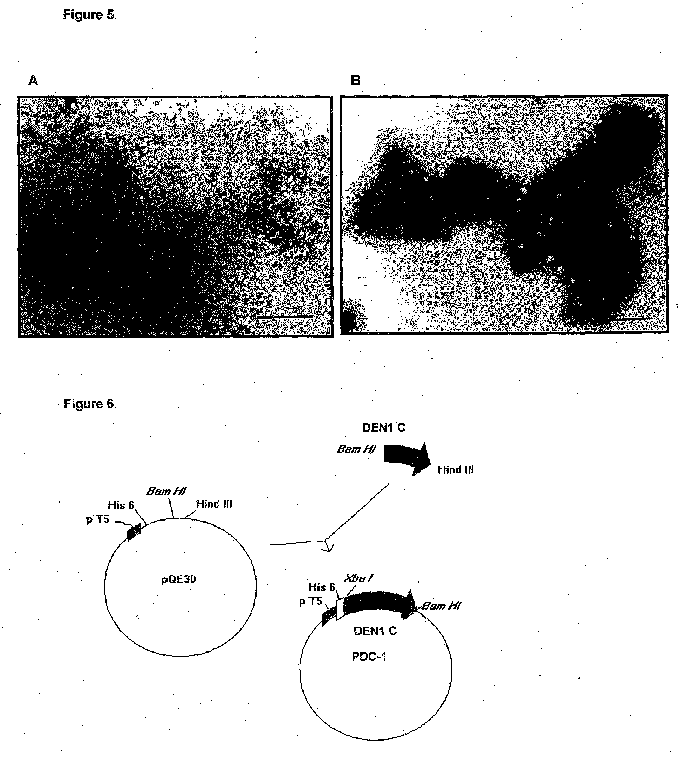

In order to reparticulate the pure capsid protein in a dimeric form, the buffer was exchanged to Hepes 25 mM, KAc 100 mM, MgAc2 1.7 mM, pH 7.4. After heating the protein and the mixture of oligonucleotides for 1 min at 37°C, they were incubated in an equal volume for 30 min at 30°C. As a negative control of the experiment, the protein was incubated without the oligonucleotides. When both preparations were observed with an electron microscope, a large quantity of particles of approximately 21 nm diameter, were observed in the sample of protein previously incubated with the mixture of oligonucleotides, while in the control sample no particles were observed (Figure 5).

### EXAMPLE 9. Immunological evaluation in mice of the purified capsid.

Five groups of 20 Balb/c mice were used. Two of them were immunized with 10 ug of the dimeric purified recombinant protein by intraperitoneal route, using aluminum hydroxide and Freund's adjuvant. Another group was immunized with 10 ug of the purified and particutated capsid protein adjuvanted with aluminum hydroxide. The soluble fraction from the rupture of *XL-1 blue* cells transformed with the plasmid pQE-30 and subjected to the same purification steps than PDC-2 was used as negative control, adjuvanted with Freund's adjuvant. The fifth group was immunized with DEN-2 virus as positive control. One month after the last dose 10 animals from each group received a dose of 100 LD₅₀ of lethal DEN-2 by intracranial inoculation and were observed for 21 days to obtain the percentages of survival. All mice in the positive control group survived, while in the negative control group all mice were sick by day 7-11 after challenge and 0% mortality was obtained. Finally, from the groups immunized with the recombinant protein, the group immunized with pure dimeric PDC-2 presented a 20% protection when immunized with aluminum hydroxide and a 40% protection when Freund's adjuvant was used. Additionally, in the group that received the reparticulated pure protein adjuvanted with aluminum hydroxide, 90% of mice were protected (Table 4).

**Table 4. Percentage of survival in mice immunized with the protein variants assayed upon challenge with the homologous lethal Dengue virus.**

| **Immunogen (adjuvant)** | **Survival percentage*** |
|---|---|
| XI-1 Blue (Freund) | 0 |
| Pure and dimeric | 20 |
| PDC-2 (Aluminum) | |
| Pure and dimeric | 40 |
| PDC-2 (Freund) | |
| Pure and reparticulated | 90 |
| PDC-2 (Aluminum) | |
| DEN-2 | 100 |

| | |
|---|---|
| *It was calculated: (# de survivors) / (total # of mice). Data of survivors were taken 21 days after challenge. | |

### EXAMPLE 10. Lymphoproliferative response

The rest of the animals from the groups immunized with the capsid protein (10 animals), either dimeric or reparticulated, adjuvanted with aluminum hydroxide, were sacrificed 15 days after the last dose. Then, their spleens were extracted and the lymphoproliferative response to DEN-2 was studied. The results in table 5 show the stimulation indexes obtained.

**Table 5. Stimulation indexes against the homologous serotype of the lymphocytes from immunized mice.**

| | **Pure and reparticulated PDC-2** | **Pure PDC-2** |
|---|---|---|
| **DEN-2**** | 10* | 4 |
| **Antigen Control***** | 1,5 | 1,2 |
| **PHA****** | 7 | 8 |

| | | |
|---|---|---|
| * Stimulation index: quotient average of counts / minutes of samples between counts / minutes of the ADN spontaneous synthesis control. ** Preparation of DEN-2 infected mice brain. *** Rreparation of not infected mice brain. **** Phytohemaglutinina Mitogen (Positive Control). | | |

### EXAMPLE 11. Immunological evaluation of the mixture formed by PD5 and PDC-2

Twenty animals were inoculated with the mixture of 10 ug of the particulated pure capsid protein and 20 ug of protein PD5 (Sequence No. 23) in three doses spaced fifteen days apart. A group immunized with 10 ug of the pure capsid protein, a group immunized with 20 ug of protein PD5 mixed with the equivalent volume of PDC-2 but obtained from a negative control run, and a group immunized with protein P64k, the carrier protein present in the construction of PD5, were used as controls. In all cases, aluminum hydroxide was used as adjuvant.

Fifteen days after the last dose, the animals were bled and the sera tested for antiviral antibodies by ELISA. As shown in tables 6 and 7, the group immunized with the mixture developed serotype-specific antibodies with titers higher than those of the group immunized only with protein PD5 and, at the same time, titers in these two groups were higher than those in the group immunized with protein PDC-2, where no Abs against DEN-2 virus were detected. On the other hand, 10 additional animals were taken from each group for lymphoproliferation assays. The cells from the spleens of these animals were extracted and stimulated with the infective DEN-2 virus. As shown in table 8, in the group immunized with the mixture the stimulation indexes were higher than those in the group immunized with the capsid protein only. The lowest stimulation indexes were obtained in the group immunized with protein PD5.

**Table 6. Antibody titers against DEN-2 virus in sera obtained after the immunization.**

| **Mouse** | **Groups inoculated with:** | | | |
|---|---|---|---|---|
| | **PDC-2** | **PDC-2/PD5** | **PD5** | **P64k** |
| 1 | < 1: 100 | < 1: 128000 | < 1: 64000 | < 1: 100 |
| 2 | < 1: 100 | < 1: 320000 | < 1: 32000 | < 1: 100 |
| 3 | < 1: 100 | < 1: 320000 | < 1: 64000 | < 1: 100 |
| 4 | < 1: 100 | < 1: 320000 | < 1: 16000 | < 1: 100 |
| 5 | < 1: 100 | < 1: 64000 | < 1: 64000 | < 1: 100 |
| 6 | < 1: 100 | < 1: 128000 | < 1: 128000 | < 1: 100 |
| 7 | < 1: 100 | < 1: 64000 | < 1: 64000 | < 1: 100 |
| 8 | < 1: 100 | < 1: 128000 | < 1: 32000 | < 1: 100 |
| 9 | < 1: 100 | < 1: 320000 | < 1: 64000 | < 1: 100 |
| 10 | < 1: 100 | < 1: 320000 | < 1: 32000 | < 1: 100 |

**Table 7. Determination of the serotype-specifcity of the antibodies contained in the mixtures of the sera obtained from each group.**

| | **Groups inoculated with:** | | | |
|---|---|---|---|---|
| **Viral Antigen** | **PDC-2** | **PDC-2/PD5** | **PD5** | **P64k** |
| DEN-1 | < 1: 100 | <1:200 | <1:200 | < 1: 100 |
| DEN-2 | < 1: 100 | 1: 320 000 | 1:64000 | < 1: 100 |
| DEN-3 | < 1: 100 | <1:200 | <1:200 | < 1: 100 |
| DEN-4 | < 1: 100 | <1:200 | <1:200 | < 1: 100 |

**Table 8. Stimulation indexes against the homologous serotype of the lymphocytes from immunized mice.**

| | **PDC-2** | **PDC-2/PDS** | **PD5** | **P64k** |
|---|---|---|---|---|
| **DEN-2**** | 9* | 11 | 2,1 | 1,1 |
| **Antigen*** Control (-)** | 1,3 | 1,6 | 1,5 | 1,2 |
| **PHA****** | 7,5 | 7,3 | 7,9 | 8 |

| | | | | |
|---|---|---|---|---|
| * Stimulation index: quotient average of counts / minutes of samples between counts / minutes of the ADN spontaneous synthesis control. ** Preparation of DEN-2 infected mice brain. *** Preparation of not infected mice brain. **** Phytohemaglutinina Mitogen (positive control). | | | | |

### EXAMPLE 12. CD8 depletion studies.

The reparticulated and the dimeric capsid proteins were inoculated in Balb/c mice to obtain some evidence of induction of cellular immune response. A preparation obtained from cells transformed with the plasmid used to generate pDC-2, and by a purification process similar to the one used for the protein PDC-2, was employed as a negative control.

Three doses of the protein (20ug) were administered to groups of 20 animals, using aluminum hydroxide as adjuvant. One month after the last dose, 1 mg of a rat anti-mouse CD8 mAb, able to deplete the cells of the mouse immune system containing this marker was administered to half of the animals of each group. On the next day, all the animals were challenged with 100 LD₅₀ (Median Lethal doses) of DEN-2 virus. They were observed for the onset of signs of disease and deaths were recorded.

In the case of the immunized non-treated groups, 20 and 80% protection was obtained in the groups immunized with the dimeric and the reparticulated capsid, respectively. Parallely, in the treated groups the percentage of protection was lower than in the non-treated groups: 0% protection for the dimeric PDC-2 and 10% protection for the reparticulated protein. In the case of the negative control group no protection was obtained in either the treated or the non-treated animals.

**Table 9. Challenge assay with DEN-2 lethal virus in the animals immunized with variants of the recombinant capsid**

| **Groups** | ***Survival percentages in mice treated with the anti-CD8 mAb** | **Survival percentages in mice non treated with the anti-CD8 mAb** |
|---|---|---|
| PCD12 reparticulated | 10 | 80 |
| PCD12 non-particulated | 0 | 20 |
| Control (-) | 0 | 0 |

| | | |
|---|---|---|
| * It was calculated: (# de survivors) / (total # of mice). Data of survivors were taken 21 days after challenge. | | |

### EXAMPLE 13: Obtaining and semipurification of the DEN-1 protein.

The nucleotide sequence that codes for amino acids 1 to 100 of the capsid protein of DEN-1 virus (Sequence No. 7), was amplified with the oligonucleotides identified in the sequence list as Sequence No. 8 and Sequence No. 10 from the DEN-1 viral strain. The vector was generated by digestion *BamHI*/*HindIII* of the plasmid pQE-30, which contains the phage T5 promoter and a 6 histidine tail in the N-terminal region (Sequence No. 6). Upon ligation, the recombinants were analyzed by restriction and the positives clones were sequenced to check the junctions. Competent cells XL-1 Blue (Hanahan D. 1983. Studies on transformation of Escherichia coli with plasmids. J. Mol. Biol. 166:557-580) were transformed with the selected clone called pDC-1 (Figure 6 y Sequence No. 10). The *E. coli* strains transformed were cultivated in LB supplemented with Ampicilline 50 µg/mL for 10 h at 37°C. Isopropyl-*B*-D-thiogalactopyranoside (IPTG) to a final concentration of 1 mM was used for the induction of the promoter. Upon growing the colony, an SDS-PAGE of the cellular lysate was done. As a result a 15-kDA band was obtained. The protein was recognized by an anti-DEN-1 HMAF. This protein was denominated PDC-1 (Sequence No. 11)

### EXAMPLE 14. Semipurification and characterization of PDC-1.

The biomass obtained from the *E. coli* strain transformed with pDC-1 and grown at 37°C was disrupted by French press. The recombinant protein was obtained equally distributed between the soluble and insoluble fractions. From the soluble fraction an anionic interchange chromatography was done, using a Q *Sepharose FF* column and the buffer Tris 10mM pH 8. The protein in the non-absorbed fraction was obtained with 45% of purity, and was used to the immunological studies.

### EXAMPLE 15: Immunological evaluation of semipurified PDC-1.

Two groups of 30 Balb/c mice were used. One of them was immunized with 10 ug of the recombinant protein by intraperitoneal route, using the aluminum hydroxide as adjuvant. The soluble fraction resulting from the rupture of the pQE-30-transformed cells adjuvanted with aluminum hydroxide was used as negative control. A part of the animals (10 mice) were bled 15 days after the third dose and the antibody titers against DEN-1 were determined by ELISA. After the immunization with the recombinant protein, no antiviral antibody titers were obtained.

**Table 10. Antibodies titers against DEN-1 virus from sera obtained after the immunization with the semipurified PDC-1.**

| **Mouse** | **Anti- DEN-1 ELISA titers** | |
|---|---|---|
| | **XL-1 blue Control (-)** | **PDC-1** |
| 1 | < 1: 100 | < 1: 100 |
| 2 | < 1: 100 | < 1: 100 |
| 3 | < 1: 100 | < 1: 100 |
| 4 | < 1: 100 | < 1: 100 |
| 5 | < 1: 100 | < 1: 100 |
| 6 | < 1: 100 | < 1: 100 |
| 7 | < 1: 100 | < 1: 100 |
| 8 | < 1: 100 | < 1: 100 |
| 9 | < 1: 100 | < 1: 100 |
| 10 | < 1: 100 | < 1: 100 |

### EXAMPLE 16. Protection assay

For the evaluation of the protection conferred to mice against challenge with lethal homologous DEN virus by the immunization with the described variants, 10 mice were used from each of the groups immunized with the recombinant protein adsorbed in aluminum hydroxide and with the control preparation. Each animal received a dose of 100 LD₅₀ of lethal DEN-1 by intracranial inoculation and was observed for 21 days to obtain the percentages of lethality in terms of death by viral encephalitis. As a positive control, a group of 10 mice immunized with infective DEN-1 virus (10⁴ pfu) was used. All mice in the positive control group survived, while in the negative control group all mice were sick at day 7-11 after challenge and 100% mortality was obtained at day 21. Finally, the group immunized with the recombinant protein PDC-1 presented 50% of protection (Table 11).

**Table 11. Percentage of survival in mice immunized with the protein variants assayed upon challenge with the homologous lethal DEN virus.**

| Immunogen | Survival percentages* |
|---|---|
| XL-1 blue (Control -) | 0 |
| DEN-1 (Control +) | 100 |
| PDC-1 | 50 |

| | |
|---|---|
| * It was calculated: (# de survivors) / (total # of mice). Data of survivors were taken 21 days afte challenge. | |

### Example 17. Lymphoproliferative response

The rest of the animals of the group immunized with the protein PDC-1 were sacrificed 15 days after the last dose. Then, their spleens were extracted and the lymphoproliferative response to DEN-1 was studied. The results in table 12 show the stimulation indexes obtained.

**Table 12. Stimulation indexes against the homologous serotype of the lymphocytes from immunized mice.**

| | PDC-1 aluminum hydroxide |
|---|---|
| DEN1** | 8* |
| Control Antigen*** | 1.5 |
| PHA**** | 7 |

| | |
|---|---|
| * Stimulation index: quotient average of counts / minutes of samples between counts / minutes of the ADN spontaneous synthesis control. ** Preparation of DEN-2 infected mice brain. *** Preparation of not infected mice brain. **** Phytohemaglutinina Mitogen (Positive Control). | |

### EXAMPLE 18. Cloning and expression of PDC-2 DomIII.

The nucleotide sequence that codes for amino acids 286 to 426 of the envelope protein from DEN-2 (Sequence No. 12), corresponding to the region of the domain III of the protein, was amplified with the oligonucleotides identified in the sequence list as Sequence No. 13 and Sequence No. 14 from the DEN-2 virus strain genotype Jamaica (Deubel V., Kinney R.M., Trent D.W. Nucleotide sequence and deduced amino acid sequence of the nonstructural proteins of Dengue type 2 virus, Jamaica genotype: Comparative analysis of the full-length genome. Virology 1988.165:234-244).

The vector was created by digestion of the plasmid pDC-2 with *BamHI*/*BamHI*, which contains the phage T5 promoter, a 6-histidine tail in the N-terminal region and the region corresponding to 100 amino acids of the capsid protein of DEN-2 virus. Upon ligation, the potential recombinants were analyzed by restriction enzyme digestion and positive clones were sequenced to check up the junctions. Finally the clone selected was named pDC-2 Dom III (Sequence No 15).

Competent cells XL-1 Blue (Hanahan D. 1983. Studies on transformation of Escherichia coli with plasmids. J. Mol. Biol. 166:557-580) were transformed with the selected clone called pDC-2 DomIII. The *E. coli* strains transformed were cultivated in LB supplemented with Ampicilline 50 µg/mL for 10 h at 37°C. Isopropyl-*B*-D-thiogalactopyranoside (IPTG) to a final concentration of 1mM was used to the induction of the promoter. Upon growing the colony, an SDS-PAGE of the cellular lysate was done. As a result, a 30-kDA band was obtained. The protein was recognized by an anti-DEN-2 HMAF. This protein was denominated PDC-2 Dom III (Sequence No. 16).

### EXAMPLE 19. Semipurification and characterization of PDC-2 Dom III.

The biomass obtained from the *E. coli* strain transformed with pDC-2 DomIII and grown at 37°C was disrupted by French press. The recombinant protein was obtained equally distributed between the soluble and insoluble fractions. From the soluble fraction an anionic interchange chromatography was done, using a Q *Sepharose* FF column and the buffer Tris 10mM pH 8. The protein in the non-absorbed fraction was obtained with 40% of purity, and was used to the immunological studies (Fig. 2).

### EXAMPLE 20. Immunological evaluation in mice of the semipurified PDC-2 Dom III.

Five groups of 30 Balb/c mice were used. One of the groups was immunized with 10 ug of the recombinant protein by intraperitoneal route, using aluminum hydroxide as adjuvant. The soluble fraction resulting from the rupture of the *XL-1 Blue* cells transformed with the plasmid pQE-30 was used as negative control, adjuvanted with aluminum hydroxide. Another two groups were included as controls. One of them was immunized with the protein PDC-2 and the other with the protein PD5 (this protein contains the domain III region of the envelope protein of DEN-2 virus). Ten animals from each group were bled 15 days after the third dose and the antibody titers against DEN-2 were determined by ELISA. As shown in Tables 13 and 14, the group immunized with PDC-2 Dom III developed high titers of serotype-specific antibodies against DEN-2, higher than those induced by the protein PD5. These results demonstrate that the genetic combination with the capsid protein enhances the antiviral immune response elicited by the domain III of the envelope protein.

**Table 13. Antibodies titers against DEN-2 virus from sera obtained after the immunization with the Dom III-capsid protein.**

| **Mouse** | **Groups immunized with:** | | | |
|---|---|---|---|---|
| | **PDC-2** | **PDC-2 Dom III** | **PD5** | **P64k** |
| 1 | < 1: 100 | < 1: 320000 | < 1: 32000 | < 1: 100 |
| 2 | < 1: 100 | < 1: 640000 | < 1: 32000 | < 1: 100 |
| 3 | < 1: 100 | < 1: 640000 | < 1: 64000 | < 1: 100 |
| 4 | < 1: 100 | < 1: 640000 | < 1: 64000 | < 1: 100 |
| 5 | < 1: 104 | < 1: 128000 | < 1: 64000 | < 1: 100 |
| 6 | < 1: 100 | < 1: 320000 | < 1: 32000 | < 1: 100 |
| 7 | < 1: 100 | < 1: 128000 | < 1: 64000 | < 1: 100 |
| 8 | < 1: 100 | < 1: 64000 | < 1: 128000 | < 1: 100 |
| 9 | < 1: 000 | < 1: 64000 | < 1: 32000 | < 1: 100 |
| 10 | < 1: 100 | < 1: 128000 | < 1: 64000 | < 1: 100 |

**Table 14. Determination of the serotype-specificity of the antibodies contained in the mixtures of the sera obtained from each group.**

| | **Groups inoculated with:** | | | |
|---|---|---|---|---|
| **Viral Antigen** | **PDC-2** | **PDC-2 Dom III** | **PD5** | **P64k** |
| DEN-1 | < 1: 100 | <1:200 | <1:200 | < 1: 100 |
| DEN-2 | < 1: 100 | 1: 320 000 | 1:64 000 | < 1: 100 |
| DEN-3 | < 1: 100 | <1:200 | <1:200 | < 1: 100 |
| DEN-4 | < 1: 100 | <1:200 | <1:200 | < 1: 100 |

On the other hand, 10 additional animals were taken from each group for the lymphoproliferation assays. The cells from the spleens of these animals were extracted and stimulated with the infective DEN-2 virus. Table 15 shows that in the group immunized with the combination, the stimulation indexes were higher than those in the group immunized with the capsid protein only. The stimulation indexes in the group immunized with protein PD5 were the lowest.

**Table 15. Stimulation indexes against the homologous serotype of the lymphocytes from immunized mice.**

| | **PDC-2** | **PDC-2 Dom III** | **PD5** | **P64k** |
|---|---|---|---|---|
| **DEN-2**** | 9,5* | 11,6 | 2,2 | 1,2 |
| **Antigen*** Control (-)** | 1,2 | 1,1 | 1,2 | 1,6 |
| **PHA****** | 7,6 | 7,4 | 7,5 | 7,9 |

| | | | | |
|---|---|---|---|---|
| * Stimulation index: quotient average of counts / minutes of samples between counts / minutes of the ADN spontaneous synthesis control. ** Preparation of DEN-2 infected mice brain. *** Preparation of not infected mice brain. **** Phytohemaglutinina Mitogen (Positive Control). | | | | |

### SEQUENCE LISTING

<110> Center for Genetic Engineering and Biotecnology
<120> The capsid protein of Dengue virus inducer of a protective immune response and
   pharmaceutical composition.
<130> Dengue
<160> 24
<170> Patentln version 3.2
<210> 1
   <211> 24
   <212> DNA
   <213> Dengue virus
<220>
   <221> primer_bind
   <222> (1)..(24)
   <223> Sequence of the oligonucleotide BamH-I to the amplification of the
   capsid protein of dengue 2 virus
<400> 1
   cgggatccaa taaccaacga aaaa 24
<210> 2
   <211> 23
   <212> DNA
   <213> Dengue virus
<220>
   <221> primer_bind
   <222> (1)..(23)
   <223> Sequence of the oligonucleotide Hind-III to the amplification of the capsid protein of DEN-2 virus
<400> 2
   acaagctttt acctgcgtct cct 23
<210> 3
   <211> 339
   <212> DNA
   <213> Dengue virus
<220>
   <221> gene
   <222> (1)..(339)
   <223> Nucleotide sequence that codes for amino acids 1 to 113 of the capsid protein of dengue 2 virus
<400> 3
   aataaccaac gaaaaaaggc gagaagtacg cctttcaata tgctgaaacg cgagagaaac 60
   cgcgtgtcaa ctgtgcaaca gctgacaaag agattctcac ttggaatgct gcaaggacga 120
   ggaccattaa aactgttcat ggcccttgtg gcgttccttc gtttcctaac aatcccacca 180
   acagcaggga tactgaaaag atggggaacg atcaaaaaat caaaagctat caatgttttg 240
   agagggttca ggaaagagat tggaaggatg ctgaacatct tgaacaggag acgcaggaca 300
   gcaggcgtga ttattatgtt gattccaaca gcgatggcg 339
<210> 4
   <211> 339
   <212> DNA
   <213> Dengue virus
<220>
   <221> gene
   <222> (1)..(339)
   <223> Nucleotide sequence that codes for the quimeric protein PDC-2 in the plasmid pQE-30
<400> 4
   atgagaggat cgcatcacca tcaccatcac ggatccaata accaacgaaa aaaggcgaga 60
   agtacgcctt tcaatatgct gaaacgcgag agaaaccgcg tgtcaactgt gcaacagctg 120
   acaaagagat tctcacttgg aatgctgcaa ggacgaggac cattaaaact gttcatggcc 180
   cttgtggcgt tccttcgttt cctaacaatc ccaccaacag cagggatact gaaaagatgg 240
   ggaacgatca aaaaatcaaa agctatcaat gttttgagag ggttcaggaa agagattgga 300
   aggatgctga acatcttgaa caggagacgc taaaagctt 339
<210> 5
   <211> 111
   <212> PRT
   <213> Dengue virus
<220>
   <221> CHAIN
   <222> (1)..(111)
   <223> Aminoacidic sequence coresponding to the protein PDC-2.
<400> 5
   Met Arg Gly Ser His His His His His His Gly Ser Asn Asn Gln Arg
   1 5 10 15
   Lys Lys Ala Arg Ser Thr Pro Phe Asn Met Leu Lys Arg Glu Arg Asn
   20 25 30
   Arg Val Ser Thr Val Gln Gln Leu Thr Lys Arg Phe Ser Leu Gly Met
   35 40 45
   Leu Gln Gly Arg Gly Pro Leu Lys Leu Phe Met Ala Leu Val Ala Phe
   50 55 60
   Leu Arg Phe Leu Thr Ile Pro Pro Thr Ala Gly Ile Leu Lys Arg Trp
   65 70 75 80
   Gly Thr Ile Lys Lys Ser Lys Ala Ile Asn Val Leu Arg Gly Phe Arg
   85 90 95
   Lys Glu Ile Gly Arg Met Leu Asn Ile Leu Asn Arg Arg Arg Arg
   100 105 110
<210> 6
   <211> 3461
   <212> DNA
   <213> Artificial
<220>
   <223> plasmid pQE-30
<220>
   <221> misc_feature
   <223> Nucleotide sequence of the plasmid pQE-30. In italic the restriction sites BamHI/Hind III are underlined.
<400> 6
   ctcgagaaat cataaaaaat ttatttgctt tgtgagcgga taacaattat aatagattca 60
   attgtgagcg gataacaatt tcacacagaa ttcattaaag aggagaaatt aactatgaga 120
   ggatcgcatc accatcacca tcacggatcc gcatgcgagc tcggtacccc gggtcgacct 180
   gcagccaagc ttaattagct gagcttggac tcctgttgat agatccagta atgacctcag 240
   aactccatct ggatttgttc agaacgctcg gttgccgccg ggcgtttttt attggtgaga 300
   atccaagcta gcttggcgag attttcagga gctaaggaag ctaaaatgga gaaaaaaatc 360
   actggatata ccaccgttga tatatcccaa tggcatcgta aagaacattt tgaggcattt 420
   cagtcagttg ctcaatgtac ctataaccag accgttcagc tggatattac ggccttttta 480
   aagaccgtaa agaaaaataa gcacaagttt tatccggcct ttattcacat tcttgcccgc 540
   ctgatgaatg ctcatccgga atttcgtatg gcaatgaaag acggtgagct ggtgatatgg 600
   gatagtgttc acccttgtta caccgttttc catgagcaaa ctgaaacgtt ttcatcgctc 660
   tggagtgaat accacgacga tttccggcag tttctacaca tatattcgca agatgtggcg 720
   tgttacggtg aaaacctggc ctatttccct aaagggttta ttgagaatat gtttttcgtc 780
   tcagccaatc cctgggtgag tttcaccagt tttgatttaa acgtggccaa tatggacaac 840
   ttcttcgccc cgttttcacc atgggcaaat attatacgca aggcgacaag gtgctgatgc 900
   cgctggcgat tcaggttcat catgccgtct gtgatggctt ccatgtcggc agaatgctta 960
   atgaattaca acagtactgc gatgagtggc agggcggggc gtaatttttt taaggcagtt 1020
   attggtgccc ttaaacgcct ggggtaatga ctctctagct tgaggcatca aataaaacga 1080
   aaggctcagt cgaaagactg ggcctttcgt tttatctgtt gtttgtcggt gaacgctctc 1140
   ctgagtagga caaatccgcc gctctagagc tgcctcgcgc gtttcggtga tgacggtgaa 1200
   aacctctgac acatgcagct cccggagacg gtcacagctt gtctgtaagc ggatgccggg 1260
   agcagacaag cccgtcaggg cgcgtcagcg ggtgttggcg ggtgtcgggg cgcagccatg 1320
   acccagtcac gtagcgatag cggagtgtat actggcttaa ctatgcggca tcagagcaga 1380
   ttgtactgag agtgcaccat atgcggtgtg aaataccgca cagatgcgta aggagaaaat 1440
   accgcatcag gcgctcttcc gcttcctcgc tcactgactc gctgcgctcg gtctgtcggc 1500
   tgcggcgagc ggtatcagct cactcaaagg cggtaatacg gttatccaca gaatcagggg 1560
   ataacgcagg aaagaacatg tgagcaaaag gccagcaaaa ggccaggaac cgtaaaaagg 1620
   ccgcgttgct ggcgtttttc cataggctcc gcccccctga cgagcatcac aaaaatcgac 1680
   gctcaagtca gaggtggcga aacccgacag gactataaag ataccaggcg tttccccctg 1740
   gaagctccct cgtgcgctct cctgttccga ccctgccgct taccggatac ctgtccgcct 1800
   ttctcccttc gggaagcgtg gcgctttctc aatgctcacg ctgtaggtat ctcagttcgg 1860
   tgtaggtcgt tcgctccaag ctgggctgtg tgcacgaacc ccccgttcag cccgaccgct 1920
   gcgccttatc cggtaactat cgtcttgagt ccaacccggt aagacacgac ttatcgccac 1980
   tggcagcagc cactggtaac aggattagca gagcgaggta tgtaggcggt gctacagagt 2040
   tcttgaagtg gtggcctaac tacggctaca ctagaaggac agtatttggt atctgcgctc 2100
   tgctgaagcc agttaccttc ggaaaaagag ttggtagctc ttgatccggc aaacaaacca 2160
   ccgctggtag cggtggtttt tttgtttgca agcagcagat tacgcgcaga aaaaaaggat 2220
   ctcaagaaga tcctttgatc ttttctacgg ggtctgacgc tcagtggaac gaaaactcac 2280
   gttaagggat tttggtcatg agattatcaa aaaggatctt cacctagatc cttttaaatt 2340
   aaaaatgaag ttttaaatca atctaaagta tatatgagta aacttggtct gacagttacc 2400
   aatgcttaat cagtgaggca cctatctcag cgatctgtct atttcgttca tccatagctg 2460
   cctgactccc cgtcgtgtag ataactacga tacgggaggg cttaccatct ggccccagtg 2520
   ctgcaatgat accgcgagac ccacgctcac cggctccaga tttatcagca ataaaccagc 2580
   cagccggaag ggccgagcgc agaagtggtc ctgcaacttt atccgcctcc atccagtcta 2640
   ttaattgttg ccgggaagct agagtaagta gttcgccagt taatagtttg cgcaacgttg 2700
   ttgccattgc tacaggcatc gtggtgtcac gctcgtcgtt tggtatggct tcattcagct 2760
   ccggttccca acgatcaagg cgagttacat gatcccccat gttgtgcaaa aaagcggtta 2820
   gctccttcgg tcctccgatc gttgtcagaa gtaagttggc cgcagtgtta tcactcatgg 2880
   ttatggcagc actgcataat tctcttactg tcatgccatc cgtaagatgc ttttctgtga 2940
   ctggtgagta ctcaaccaag tcattctgag aatagtgtat gcggcgaccg agttgctctt 3000
   gcccggcgtc aatacgggat aataccgcgc cacatagcag aactttaaaa gtgctcatca 3060
   ttggaaaacg ttcttcgggg cgaaaactct caaggatctt accgctgttg agatccagtt 3120
   cgatgtaacc cactcgtgca cccaactgat cttcagcatc ttttactttc accagcgttt 3180
   ctgggtgagc aaaaacagga aggcaaaatg ccgcaaaaaa gggaataagg gcgacacgga 3240
   aatgttgaat actcatactc ttcctttttc aatattattg aagcatttat cagggttatt 3300
   gtctcatgag cggatacata tttgaatgta tttagaaaaa taaacaaata ggggttccgc 3360
   gcacatttcc ccgaaaagtg ccacctgacg tctaagaaac cattattatc atgacattaa 3420
   cctataaaaa taggcgtatc acgaggccct ttcgtcttca c 3461
<210> 7
   <211> 300
   <212> DNA
   <213> Dengue virus
<220>
   <221> gene
   <222> (1)..(300)
   <223> Nucleotide sequence that codes for amino acids I 1 to 100 of the capsid protein of Dengue 1 virus.
<400> 7
   atgaacaacc aacggaaaaa gacggctcga ccgtctttca atatgctgaa acgcgcgaga 60
   aaccgcgtgt caactgtttc acagttggcg aagagattct caaaaggatt gctctcaggc 120
   caaggaccca tgaaattggt gatggccttc atagcattcc taagatttct agccataccc 180
   ccaacagcag gaattttggc tagatggggc tcattcaaga agaatggagc gatcaaagtg 240
   ctacggggtt tcaagaaaga aatctcaaac atgttgaata taatgaatag aaggaaaaga 300
<210> 8
   <211> 26
   <212> DNA
   <213> Dengue virus
<220>
   <221> primer_bind
   <222> (1)..(26)
   <223> Sequence of the oligonucleotide BamH-I to the amplification of the capsid protein of DEN-1 virus.
<400> 8
   ggatccatga acaaccaacg gaaaaa 26
<210> 9
   <211> 24
   <212> DNA
   <213> Dengue virus
<220>
   <221> primer_bind
   <222> (1)..(24)
   <223> Sequence of the oligonucleotide Hind-III to the amplification of the capsid protein of DEN-1 virus
<400> 9
   aagctttctt ttccttctat tcat 24
<210> 10
   <211> 345
   <212> DNA
   <213> Dengue virus
<220>
   <221> gene
   <222> (1)..(345)
   <223> Nucleotide sequence that codes to the quimeric protein PDC-1 in the plasmid pQE-30
<400> 10
   atgagaggat cgcatcacca tcaccatcac ggatccatga acaaccaacg gaaaaagacg 60
   gctcgaccgt ctttcaatat gctgaaacgc gcgagaaacc gcgtgtcaac tgtttcacag 120
   ttggcgaaga gattctcaaa aggattgctc tcaggccaag gacccatgaa attggtgatg 180
   gccttcatag cattcctaag atttctagcc atacccccaa cagcaggaat tttggctaga 240
   tggggctcat tcaagaagaa tggagcgatc aaagtgctac ggggtttcaa gaaagaaatc 300
   tcaaacatgt tgaatataat gaatagaagg aaaagataaa agctt 345
<210> 11
   <211> 112
   <212> PRT
   <213> Dengue virus
<220>
   <221> CHAIN
   <222> (1)..(112)
   <223> Aminoacidic sequence corresponding to the protein PDC-1
<400> 11
   Met Arg Gly Ser His His His His His His Gly Ser Met Asn Asn Gln
   1 5 10 15
   Arg Lys Lys Thr Ala Arg Pro Ser Phe Asn Met Leu Lys Arg Ala Arg
   20 25 30
   Asn Arg Val Ser Thr Val Ser Gln Leu Ala Lys Arg Phe Ser Lys Gly
   35 40 45
   Leu Leu Ser Gly Gln Gly Pro Met Lys Leu Val Met Ala Phe Ile Ala
   50 55 60
   Phe Leu Arg Phe Leu Ala Ile Pro Pro Thr Ala Gly Ile Leu Ala Arg
   65 70 75 80
   Trp Gly Ser Phe Lys Lys Asn Gly Ala Ile Lys Val Leu Arg Gly Phe
   85 90 95
   Lys Lys Glu Ile Ser Asn Met Leu Asn Ile Met Asn Arg Arg Lys Arg
   100 105 110
<210> 12
   <211> 426
   <212> DNA
   <213> Dengue virus
<220>
   <221> gene
   <222> (1)..(426)
   <223> Nucleotide sequence that codes for DomB of the envelope protein of DEN-2 virus
<400> 12
   aggctgagaa tggacaaact acagctcaaa ggaatgtcat actctatgtg tacaggaaag 60
   tttaaaattg tgaaggaaat agcagaaaca caacatggaa caatagttat cagagtacaa 120
   tatgaagggg acggctctcc atgtaagatc ccttttgaga taatggattt ggaaaaaaga 180
   cacgtcttag gtcgcctgat tacagttaac ccgatcgtaa cagaaaaaga tagcccagtc 240
   aacatagaag cagaacctcc attcggagac agctacatca tcataggagt agagccggga 300
   caattgaaac tcaactggtt taagaaagga agttccatcg gccaaatgtt tgagacaaca 360
   atgagaggag cgaagagaat ggccatttta ggtgacacag cctgggattt tggaagcctg 420
   ggaggg 426
<210> 13
   <211> 21
   <212> DNA
   <213> Dengue virus
<220>
   <221> primer_bind
   <222> (1)..(21)
   <223> Sequence of the oligonucleotide 5' for the amplifcation of the region that codes for the amino acids 286 to 426 of the envelope protein of DEN-2 virus
<400> 13
   cttggatcca ttctgagaat g 21
<210> 14
   <211> 33
   <212> DNA
   <213> Dengue virus
<220>
   <221> primer_bind
   <222> (1)..(33)
   <223> Sequence of the oligonucleotide 3' for the amplification of the region that codes for the amino acids 286 to 426 of the envelope protein of DEN-2 virus
<400> 14
   tgtggatcct cctcctaggc ttccaaaatc cca 33
<210> 15
   <211> 753
   <212> DNA
   <213> Dengue virus
<220>
   <221> gene
   <222> (1)..(753)
   <223> Nucleotide sequence that codes for the quimeric protein pDC-2 Domlll
<400> 15
   catcaccatc accatcacgg atccaggctg agaatggaca aactacagct caaaggaatg 60
   tcatactcta tgtgtacagg aaagtttaaa attgtgaagg aaatagcaga aacacaacat 120
   ggaacaatag ttatcagagt acaatatgaa ggggacggct ctccatgtaa gatccctttt 180
   gagataatgg atttggaaaa aagacacgtc ttaggtcgcc tgattacagt taacccgatc 240
   gtaacagaaa aagatagccc agtcaacata gaagcagaac ctccattcgg agacagctac 300
   atcatcatag gagtagagcc gggacaattg aaactcaact ggtttaagaa aggaagttcc 360
   atcggccaaa tgtttgagac aacaatgaga ggagcgaaga gaatggccat tttaggtgac 420
   acagcctggg attttggatc cctgggagga ggatccaata accaacgaaa aaaggcgaga 480
   agtacgcctt tcaatatgct gaaacgcgag agaaaccgcg tgtcaactgt gcaacagctg 540
   acaaagagat tctcacttgg aatgctgcaa ggacgaggac cattaaaact gttcatggcc 600
   cttgtggcgt tccttcgttt cctaacaatc ccaccaacag cagggatact gaaaagatgg 660
   ggaacgatca aaaaatcaaa agctatcaat gttttgagag ggttcaggaa agagattgga 720
   aggatgctga acatcttgaa caggagacgc taa 753
<210> 16
   <211> 250
   <212> PRT
   <213> Dengue virus
<220>
   <221> CHAIN
   <222> (1)..(250)
   <223> Aminoacidic sequence of PDC-2 Dom III
<400> 16
   His His His His His His Gly Ser Arg Leu Arg Met Asp Lys Leu Gln
   1 5 10 15
   Leu Lys Gly Met Ser Tyr Ser Met Cys Thr Gly Lys Phe Lys Ile Val
   20 25 30
   Lys Glu Ile Ala Glu Thr Gln His Gly Thr Ile Val Ile Arg Val Gln
   35 40 45
   Tyr Glu Gly Asp Gly Ser Pro Cys Lys Ile Pro Phe Glu Ile Met Asp
   50 55 60
   Leu Glu Lys Arg His Val Leu Gly Arg Leu Ile Thr Val Asn Pro Ile
   65 70 75 80
   Val Thr Glu Lys Asp Ser Pro Val Asn Ile Glu Ala Glu Pro Pro Phe
   85 90 95
   Gly Asp Ser Tyr Ile Ile Ile Gly Val Glu Pro Gly Gln Leu Lys Leu
   100 105 110
   Asn Trp Phe Lys Lys Gly Ser Ser Ile Gly Gln Met Phe Glu Thr Thr
   115 120 125
   Met Arg Gly Ala Lys Arg Met Ala Ile Leu Gly Asp Thr Ala Trp Asp
   130 135 140
   Phe Gly Ser Leu Gly Gly Gly Ser Asn Asn Gln Arg Lys Lys Ala Arg
   145 150 155 160
   Ser Thr Pro Phe Asn Met Leu Lys Arg Glu Arg Asn Arg Val Ser Thr
   165 170 175
   Val Gln Gln Leu Thr Lys Arg Phe Ser Leu Gly Met Leu Gln Gly Arg
   180 185 190
   Gly Pro Leu Lys Leu Phe Met Ala Leu Val Ala Phe Leu Arg Phe Leu
   195 200 205
   Thr Ile Pro Pro Thr Ala Gly Ile Leu Lys Arg Trp Gly Thr Ile Lys
   210 215 220
   Lys Ser Lys Ala Ile Asn Val Leu Arg Gly Phe Arg Lys Glu Ile Gly
   225 230 235 240
   Arg Met Leu Asn Ile Leu Asn Arg Arg Arg
   245 250
<210> 17
   <211> 142
   <212> PRT
   <213> Dengue virus type 1
<220>
   <221> CHAIN
   <222> (1)..(142)
   <223> Aminoacidic sequence corresponding to DomB of the envelope protein of DEN-1 virus
<400> 17
   Arg Leu Lys Met Asp Lys Leu Thr Leu Lys Gly Val Ser Tyr Val Met
   1 5 10 15
   Cys Thr Gly Ser Phe Lys Leu Glu Lys Glu Val Ala Glu Thr Gln His
   20 25 30
   Gly Thr Val Leu Val Gln Val Lys Tyr Glu Gly Thr Asp Ala Pro Cys
   35 40 45
   Lys Ile Pro Phe Ser Ser Gln Asp Glu Lys Gly Val Thr Gln Asn Gly
   50 55 60
   Arg Leu Ile Thr Ala Asn Pro Ile Val Ile Asp Lys Glu Lys Pro Val
   65 70 75 80
   Asn Ile Glu Ala Glu Pro Pro Phe Gly Glu Ser Tyr Ile Val Val Gly
   85 90 95
   Ala Gly Glu Lys Ala Leu Lys Leu Ser Trp Phe Lys Lys Gly Ser Ser
   100 105 110
   Ile Gly Lys Met Phe Glu Ala Thr Ala Arg Gly Ala Arg Arg Met Ala
   115 120 125
   Ile Leu Gly Asp Thr Ala Trp Asp Phe Gly Ser Ile Gly Gly
   130 135 140
<210> 18
   <211> 142
   <212> PRT
   <213> Dengue virus type 3
<220>
   <221> CHAIN
   <222> (1)..(142)
   <223> Aminoacidic sequence corresponding to DomB of the envelope protein of DEN-3 virus
<400> 18
   Arg Leu Lys Met Asp Lys Leu Glu Leu Lys Gly Met Ser Tyr Ala Met
   1 5 10 15
   Cys Thr Asn Thr Phe Val Leu Lys Lys Glu Val Ser Glu Thr Gln His
   20 25 30
   Gly Thr Ile Leu Ile Lys Val Glu Tyr Lys Gly Glu Asp Val Pro Cys
   35 40 45
   Lys Ile Pro Phe Ser Thr Glu Asp Gly Gln Gly Lys Ala His Asn Gly
   50 55 60
   Arg Leu Ile Thr Ala Asn Pro Val Val Thr Lys Lys Glu Glu Pro Val
   65 70 75 80
   Asn Ile Glu Ala Glu Pro Pro Phe Gly Glu Ser Asn Ile Val Ile Gly
   85 90 95
   Ile Gly Asp Asn Ala Leu Lys Ile Asn Trp Tyr Lys Lys Gly Ser Ser
   100 105 110
   Ile Gly Lys Met Phe Glu Ala Thr Ala Arg Gly Ala Arg Arg Met Ala
   115 120 125
   Ile Leu Gly Asp Thr Ala Trp Asp Phe Gly Ser Val Gly Gly
   130 135 140
<210> 19
   <211> 142
   <212> PRT
   <213> Dengue virus type 4
<220>
   <221> CHAIN
   <222> (1)..(142)
   <223> Aminoacidic sequence corresponding to Dom B of the envelope protein of DEN-4 virus
<400> 19
   Lys Val Arg Met Glu Lys Leu Arg Ile Lys Gly Met Ser Tyr Thr Met
   1 5 10 15
   Cys Ser Gly Lys Phe Ser Ile Asp Lys Glu Met Ala Glu Thr Gln His
   20 25 30
   Gly Thr Thr Val Val Lys Val Lys Tyr Glu Gly Ala Gly Ala Pro Cys
   35 40 45
   Lys Val Pro Ile Glu Ile Arg Asp Val Asn Lys Glu Lys Val Val Gly
   50 55 60
   Arg Ile Ile Ser Ser Thr Pro Leu Ala Glu Asn Thr Asn Ser Val Thr
   65 70 75 80
   Asn Ile Glu Leu Glu Pro Pro Phe Gly Asp Ser Tyr Ile Val Ile Gly
   85 90 95
   Val Gly Asn Ser Ala Leu Thr Leu His Trp Phe Arg Lys Gly Ser Ser
   100 105 110
   Ile Gly Lys Met Phe Glu Ser Thr Tyr Arg Gly Ala Lys Arg Met Ala
   115 120 125
   Ile Leu Gly Glu Thr Ala Trp Asp Phe Gly Ser Val Gly Gly
   130 135 140
<210> 20
   <211> 749
   <212> PRT
   <213> Dengue virus type 1
<220>
   <221> CHAIN
   <222> (1)..(749)
   <223> Aminoacidic sequence corresponding to the protein PLH3.
<400> 20
   Met Gly His His His His His His Ala Met Val Asp Lys Arg Met Ala
   1 5 10 15
   Leu Val Glu Leu Lys Val Pro Asp Ile Gly Gly His Glu Asn Val Asp
   20 25 30
   Ile Ile Ala Val Glu Val Asn Val Gly Asp Thr Ile Ala Val Asp Asp
   35 40 45
   Thr Leu Ile Thr Leu Asp Leu Glu Met Asp Val Pro Ala Glu Val Ala
   50 55 60
   Gly Val Val Lys Glu Val Lys Val Lys Val Gly Asp Lys Ile Ser Glu
   65 70 75 80
   Gly Gly Leu Ile Val Val Val Glu Ala Glu Gly Thr Ala Ala Ala Pro
   85 90 95
   Lys Ala Glu Ala Ala Ala Ala Pro Ala Gln Glu Ala Pro Lys Ala Ala
   100 105 110
   Ala Pro Ala Pro Gln Ala Ala Gln Phe Gly Gly Ser Ala Asp Ala Glu
   115 120 125
   Tyr Asp Val Val Val Leu Gly Gly Gly Pro Gly Gly Tyr Ser Ala Ala
   130 135 140
   Phe Ala Ala Ala Asp Glu Gly Leu Lys Val Ala Ile Val Glu Arg Tyr
   145 150 155 160
   Lys Thr Leu Gly Gly Val Cys Leu Asn Val Gly Cys Ile Pro Ser Lys
   165 170 175
   Ala Leu Leu His Asn Ala Ala Val Ile Asp Glu Val Arg His Leu Ala
   180 185 190
   Ala Asn Gly Ile Lys Tyr Pro Glu Pro Glu Leu Asp Ile Asp Met Leu
   195 200 205
   Arg Ala Tyr Lys Asp Gly Val Val Ser Arg Leu Thr Gly Gly Leu Ala
   210 215 220
   Gly Met Ala Lys Ser Arg Lys Val Asp Val Ile Gln Gly Asp Gly Gln
   225 230 235 240
   Phe Leu Asp Pro His His Leu Glu Val Ser Leu Thr Ala Gly Asp Ala
   245 250 255
   Tyr Glu Gln Ala Ala Pro Thr Gly Glu Lys Lys Ile Val Ala Phe Lys
   260 265 270
   Asn Cys Ile Ile Ala Ala Gly Ser Arg Val Thr Lys Leu Pro Phe Ile
   275 280 285
   Pro Glu Asp Pro Arg Ile Ile Asp Ser Ser Gly Ala Leu Ala Leu Lys
   290 295 300
   Glu Val Pro Gly Lys Leu Leu Ile Ile Gly Gly Gly Ile Ile Gly Leu
   305 310 315 320
   Glu Met Gly Thr Val Tyr Ser Thr Leu Gly Ser Arg Leu Asp Val Val
   325 330 335
   Glu Met Met Asp Gly Leu Met Gln Gly Ala Asp Arg Asp Leu Val Lys
   340 345 350
   Val Trp Gln Lys Gln Asn Glu Tyr Arg Phe Asp Asn Ile Met Val Asn
   355 360 365
   Thr Lys Thr Val Ala Val Glu Pro Lys Glu Asp Gly Val Tyr Val Thr
   370 375 380
   Phe Glu Gly Ala Asn Ala Pro Lys Glu Pro Gln Arg Tyr Asp Ala Val
   385 390 395 400
   Leu Val Ala Ala Gly Arg Ala Pro Asn Gly Lys Leu Ile Ser Ala Glu
   405 410 415
   Lys Ala Gly Val Ala Val Thr Asp Arg Gly Phe Ile Glu Val Asp Lys
   420 425 430
   Gln Met Arg Thr Asn Val Pro His Ile Tyr Ala Ile Gly Asp Ile Val
   435 440 445
   Gly Gln Pro Met Leu Ala His Lys Ala Val His Glu Gly His Val Ala
   450 455 460
   Ala Glu Asn Cys Ala Gly His Lys Ala Tyr Phe Asp Ala Arg Val Ile
   465 470 475 480
   Pro Gly Val Ala Tyr Thr Ser Pro Glu Val Ala Trp Val Gly Glu Thr
   485 490 495
   Glu Leu Ser Ala Lys Ala Ser Gly Arg Lys Ile Thr Lys Ala Asn Phe
   500 505 510
   Pro Trp Ala Ala Ser Gly Arg Ala Ile Ala Asn Gly Cys Asp Lys Pro
   515 520 525
   Phe Thr Lys Leu Ile Phe Asp Ala Glu Thr Gly Arg Ile Ile Gly Gly
   530 535 540
   Gly Ile Val Gly Pro Asn Gly Gly Asp Met Ile Gly Glu Val Cys Leu
   545 550 555 560
   Ala Ile Glu Met Gly Cys Asp Ala Ala Asp Ile Gly Lys Thr Ile His
   565 570 575
   Pro His Pro Thr Leu Gly Glu Ser Ile Gly Met Ala Ala Glu Val Ala
   580 585 590
   Leu Gly Thr Cys Thr Asp Leu Pro Pro Gln Lys Lys Lys Gly Ser Arg
   595 600 605
   Leu Lys Met Asp Lys Leu Thr Leu Lys Gly Val Ser Tyr Val Met Cys
   610 615 620
   Thr Gly Ser Phe Lys Leu Glu Lys Glu Val Ala Glu Thr Gln His Gly
   625 630 635 640
   Thr Val Leu Val Gln Val Lys Tyr Glu Gly Thr Asp Ala Pro Cys Lys
   645 650 655
   Ile Pro Phe Ser Ser Gln Asp Glu Lys Gly Val Thr Gln Asn Gly Arg
   660 665 670
   Leu Ile Thr Ala Asn Pro Ile Val Ile Asp Lys Glu Lys Pro Val Asn
   675 680 685
   Ile Glu Ala Glu Pro Pro Phe Gly Glu Ser Tyr Ile Val Val Gly Ala
   690 695 700
   Gly Glu Lys Ala Leu Lys Leu Ser Trp Phe Lys Lys Gly Ser Ser Ile
   705 710 715 720
   Gly Lys Met Phe Glu Ala Thr Ala Arg Gly Ala Arg Arg Met Ala Ile
   725 730 735
   Leu Gly Asp Thr Ala Trp Asp Phe Gly Ser Ile Gly Gly
   740 745
<210> 21
   <211> 749
   <212> PRT
   <213> Dengue virus type 3
<220>
   <221> CHAIN
   <222> (1)..(749)
   <223> Aminoacidic sequence corresponding to the protein PAZ3.
<400> 21
   Met Gly His His His His His His Ala Met Val Asp Lys Arg Met Ala
   1 5 10 15
   Leu Val Glu Leu Lys Val Pro Asp Ile Gly Gly His Glu Asn Val Asp
   20 25 30
   Ile Ile Ala Val Glu Val Asn Val Gly Asp Thr Ile Ala Val Asp Asp
   35 40 45
   Thr Leu Ile Thr Leu Asp Leu Glu Met Asp Val Pro Ala Glu Val Ala
   50 55 60
   Gly Val Val Lys Glu Val Lys Val Lys Val Gly Asp Lys Ile Ser Glu
   65 70 75 80
   Gly Gly Leu Ile Val Val Val Glu Ala Glu Gly Thr Ala Ala Ala Pro
   85 90 95
   Lys Ala Glu Ala Ala Ala Ala Pro Ala Gln Glu Ala Pro Lys Ala Ala
   100 105 110
   Ala Pro Ala Pro Gln Ala Ala Gln Phe Gly Gly Ser Ala Asp Ala Glu
   115 120 125
   Tyr Asp Val Val Val Leu Gly Gly Gly Pro Gly Gly Tyr Ser Ala Ala
   130 135 140
   Phe Ala Ala Ala Asp Glu Gly Leu Lys Val Ala Ile Val Glu Arg Tyr
   145 150 155 160
   Lys Thr Leu Gly Gly Val Cys Leu Asn Val Gly Cys Ile Pro Ser Lys
   165 170 175
   Ala Leu Leu His Asn Ala Ala Val Ile Asp Glu Val Arg His Leu Ala
   180 185 190
   Ala Asn Gly Ile Lys Tyr Pro Glu Pro Glu Leu Asp Ile Asp Met Leu
   195 200 205
   Arg Ala Tyr Lys Asp Gly Val Val Ser Arg Leu Thr Gly Gly Leu Ala
   210 215 220
   Gly Met Ala Lys Ser Arg Lys Val Asp Val Ile Gln Gly Asp Gly Gln
   225 230 235 240
   Phe Leu Asp Pro His His Leu Glu Val Ser Leu Thr Ala Gly Asp Ala
   245 250 255
   Tyr Glu Gln Ala Ala Pro Thr Gly Glu Lys Lys Ile Val Ala Phe Lys
   260 265 270
   Asn Cys Ile Ile Ala Ala Gly Ser Arg Val Thr Lys Leu Pro Phe Ile
   275 280 285
   Pro Glu Asp Pro Arg Ile Ile Asp Ser Ser Gly Ala Leu Ala Leu Lys
   290 295 300
   Glu Val Pro Gly Lys Leu Leu Ile Ile Gly Gly Gly Ile Ile Gly Leu
   305 310 315 320
   Glu Met Gly Thr Val Tyr Ser Thr Leu Gly Ser Arg Leu Asp Val Val
   325 330 335
   Glu Met Met Asp Gly Leu Met Gln Gly Ala Asp Arg Asp Leu Val Lys
   340 345 350
   Val Trp Gln Lys Gln Asn Glu Tyr Arg Phe Asp Asn Ile Met Val Asn
   355 360 365
   Thr Lys Thr Val Ala Val Glu Pro Lys Glu Asp Gly Val Tyr Val Thr
   370 375 380
   Phe Glu Gly Ala Asn Ala Pro Lys Glu Pro Gln Arg Tyr Asp Ala Val
   385 390 395 400
   Leu Val Ala Ala Gly Arg Ala Pro Asn Gly Lys Leu Ile Ser Ala Glu
   405 410 415
   Lys Ala Gly Val Ala Val Thr Asp Arg Gly Phe Ile Glu Val Asp Lys
   420 425 430
   Gln Met Arg Thr Asn Val Pro His Ile Tyr Ala Ile Gly Asp Ile Val
   435 440 445
   Gly Gln Pro Met Leu Ala His Lys Ala Val His Glu Gly His Val Ala
   450 455 460
   Ala Glu Asn Cys Ala Gly His Lys Ala Tyr Phe Asp Ala Arg Val Ile
   465 470 475 480
   Pro Gly Val Ala Tyr Thr Ser Pro Glu Val Ala Trp Val Gly Glu Thr
   485 490 495
   Glu Leu Ser Ala Lys Ala Ser Gly Arg Lys Ile Thr Lys Ala Asn Phe
   500 505 510
   Pro Trp Ala Ala Ser Gly Arg Ala Ile Ala Asn Gly Cys Asp Lys Pro
   515 520 525
   Phe Thr Lys Leu Ile Phe Asp Ala Glu Thr Gly Arg Ile Ile Gly Gly
   530 535 540
   Gly Ile Val Gly Pro Asn Gly Gly Asp Met Ile Gly Glu Val Cys Leu
   545 550 555 560
   Ala Ile Glu Met Gly Cys Asp Ala Ala Asp Ile Gly Lys Thr Ile His
   565 570 575
   Pro His Pro Thr Leu Gly Glu Ser Ile Gly Met Ala Ala Glu Val Ala
   580 585 590
   Leu Gly Thr Cys Thr Asp Leu Pro Pro Gln Lys Lys Lys Gly Ser Arg
   595 600 605
   Leu Lys Met Asp Lys Leu Lys Leu Lys Gly Met Ser Tyr Ala Met Cys
   610 615 620
   Leu Asn Thr Phe Val Leu Lys Lys Glu Val Ser Glu Thr Gln His Gly
   625 630 635 640
   Thr Ile Leu Ile Lys Val Glu Tyr Lys Gly Glu Asp Ala Pro Cys Lys
   645 650 655
   Ile Pro Phe Ser Thr Glu Asp Gly Gln Gly Lys Ala His Asn Gly Arg
   660 665 670
   Leu Ile Thr Ala Asn Pro Val Val Thr Lys Lys Glu Glu Pro Val Asn
   675 680 685
   Ile Glu Ala Glu Pro Pro Phe Gly Glu Ser Asn Ile Val Ile Gly Ile
   690 695 700
   Gly Asp Lys Ala Leu Lys Ile Asn Trp Tyr Arg Lys Gly Ser Ser Ile
   705 710 715 720
   Gly Lys Met Phe Glu Ala Thr Ala Arg Gly Ala Arg Arg Met Ala Ile
   725 730 735
   Leu Gly Asp Thr Ala Trp Asp Phe Gly Ser Val Gly Gly
   740 745
<210> 22
   <211> 749
   <212> PRT
   <213> Dengue virus type 4
<220>
   <221> CHAIN
   <222> (1)..(749)
   <223> Aminoacidic sequence corresponding to PID3.
<400> 22
   Met Gly His His His His His His Ala Met Val Asp Lys Arg Met Ala
   1 5 10 15
   Leu Val Glu Leu Lys Val Pro Asp Ile Gly Gly His Glu Asn Val Asp
   20 25 30
   Ile Ile Ala Val Glu Val Asn Val Gly Asp Thr Ile Ala Val Asp Asp
   35 40 45
   Thr Leu Ile Thr Leu Asp Leu Glu Met Asp Val Pro Ala Glu Val Ala
   50 55 60
   Gly Val Val Lys Glu Val Lys Val Lys Val Gly Asp Lys Ile Ser Glu
   65 70 75 80
   Gly Gly Leu Ile Val Val Val Glu Ala Glu Gly Thr Ala Ala Ala Pro
   85 90 95
   Lys Ala Glu Ala Ala Ala Ala Pro Ala Gln Glu Ala Pro Lys Ala Ala
   100 105 110
   Ala Pro Ala Pro Gln Ala Ala Gln Phe Gly Gly Ser Ala Asp Ala Glu
   115 120 125
   Tyr Asp Val Val Val Leu Gly Gly Gly Pro Gly Gly Tyr Ser Ala Ala
   130 135 140
   Phe Ala Ala Ala Asp Glu Gly Leu Lys Val Ala Ile Val Glu Arg Tyr
   145 150 155 160
   Lys Thr Leu Gly Gly Val Cys Leu Asn Val Gly Cys Ile Pro Ser Lys
   165 170 175
   Ala Leu Leu His Asn Ala Ala Val Ile Asp Glu Val Arg His Leu Ala
   180 185 190
   Ala Asn Gly Ile Lys Tyr Pro Glu Pro Glu Leu Asp Ile Asp Met Leu
   195 200 205
   Arg Ala Tyr Lys Asp Gly Val Val Ser Arg Leu Thr Gly Gly Leu Ala
   210 215 220
   Gly Met Ala Lys Ser Arg Lys Val Asp Val Ile Gln Gly Asp Gly Gln
   225 230 235 240
   Phe Leu Asp Pro His His Leu Glu Val Ser Leu Thr Ala Gly Asp Ala
   245 250 255
   Tyr Glu Gln Ala Ala Pro Thr Gly Glu Lys Lys Ile Val Ala Phe Lys
   260 265 270
   Asn Cys Ile Ile Ala Ala Gly Ser Arg Val Thr Lys Leu Pro Phe Ile
   275 280 285
   Pro Glu Asp Pro Arg Ile Ile Asp Ser Ser Gly Ala Leu Ala Leu Lys
   290 295 300
   Glu Val Pro Gly Lys Leu Leu Ile Ile Gly Gly Gly Ile Ile Gly Leu
   305 310 315 320
   Glu Met Gly Thr Val Tyr Ser Thr Leu Gly Ser Arg Leu Asp Val Val
   325 330 335
   Glu Met Met Asp Gly Leu Met Gln Gly Ala Asp Arg Asp Leu Val Lys
   340 345 350
   Val Trp Gln Lys Gln Asn Glu Tyr Arg Phe Asp Asn Ile Met Val Asn
   355 360 365
   Thr Lys Thr Val Ala Val Glu Pro Lys Glu Asp Gly Val Tyr Val Thr
   370 375 380
   Phe Glu Gly Ala Asn Ala Pro Lys Glu Pro Gln Arg Tyr Asp Ala Val
   385 390 395 400
   Leu Val Ala Ala Gly Arg Ala Pro Asn Gly Lys Leu Ile Ser Ala Glu
   405 410 415
   Lys Ala Gly Val Ala Val Thr Asp Arg Gly Phe Ile Glu Val Asp Lys
   420 425 430
   Gln Met Arg Thr Asn Val Pro His Ile Tyr Ala Ile Gly Asp Ile Val
   435 440 445
   Gly Gln Pro Met Leu Ala His Lys Ala Val His Glu Gly His Val Ala
   450 455 460
   Ala Glu Asn Cys Ala Gly His Lys Ala Tyr Phe Asp Ala Arg Val Ile
   465 470 475 480
   Pro Gly Val Ala Tyr Thr Ser Pro Glu Val Ala Trp Val Gly Glu Thr
   485 490 495
   Glu Leu Ser Ala Lys Ala Ser Gly Arg Lys Ile Thr Lys Ala Asn Phe
   500 505 510
   Pro Trp Ala Ala Ser Gly Arg Ala Ile Ala Asn Gly Cys Asp Lys Pro
   515 520 525
   Phe Thr Lys Leu Ile Phe Asp Ala Glu Thr Gly Arg Ile Ile Gly Gly
   530 535 540
   Gly Ile Val Gly Pro Asn Gly Gly Asp Met Ile Gly Glu Val Cys Leu
   545 550 555 560
   Ala Ile Glu Met Gly Cys Asp Ala Ala Asp Ile Gly Lys Thr Ile His
   565 570 575
   Pro His Pro Thr Leu Gly Glu Ser Ile Gly Met Ala Ala Glu Val Ala
   580 585 590
   Leu Gly Thr Cys Thr Asp Leu Pro Pro Gln Lys Lys Lys Gly Ser Lys
   595 600 605
   Val Arg Met Glu Lys Leu Arg Ile Lys Gly Met Ser Tyr Thr Met Cys
   610 615 620
   Ser Gly Lys Phe Ser Ile Asp Lys Glu Met Ala Glu Thr Gln His Gly
   625 630 635 640
   Thr Thr Val Val Lys Val Lys Tyr Glu Gly Ala Gly Ala Pro Cys Lys
   645 650 655
   Val Pro Ile Glu Ile Arg Asp Val Asn Lys Glu Lys Val Val Gly Arg
   660 665 670
   Ile Ile Ser Ser Thr Pro Leu Ala Glu Asn Thr Asn Ser Val Thr Asn
   675 680 685
   Ile Glu Leu Glu Pro Pro Phe Gly Asp Ser Tyr Ile Val Ile Gly Val
   690 695 700
   Gly Asn Ser Ala Leu Thr Leu His Trp Phe Arg Lys Gly Ser Ser Ile
   705 710 715 720
   Gly Lys Met Phe Glu Ser Thr Tyr Arg Gly Ala Lys Arg Met Ala Ile
   725 730 735
   Leu Gly Glu Thr Ala Trp Asp Phe Gly Ser Val Gly Gly
   740 745
<210> 23
   <211> 750
   <212> PRT
   <213> Dengue virus type 2
<220>
   <221> CHAIN
   <222> (1)..(750)
   <223> Aminoacidic sequence corresponding to PLL3.
<400> 23
<210> 24
   <211> 142
   <212> PRT
   <213> Dengue virus type 2
<220>
   <221> CHAIN
   <222> (1)..(142)
   <223> Aminoacidic sequence corresponding to the DomB of the envelope protein of DEN-2 virus.
<400> 24 Lic. Manuel Selman-Housein Sosa.
   Legal Representative.
   CIGB.

## Claims

1. A pharmaceutical preparation **characterized by** being a vaccine, said vaccine containing as immunizing antigen the capsid protein of one or several Dengue virus serotypes in recombinant form for use in a method of inducing a protective immune response against Dengue viruses in the receiving organism by immunoenhancement, wherein said immunoenhancement is the enhanced induction of antiviral serotype-specific antibodies in a subject by a heterologous Dengue antigen.

2. Pharmaceutical preparation according to claim 1 for use in the method according to claim 1, wherein said heterologous Dengue antigen is the domain III of the envelope protein of a Dengue virus.

3. A pharmaceutical preparation according to claim 1 for use in the method according to claim 1, **characterized by** containing the capsid protein of Dengue virus 1, 2, 3 or 4, alone, mixed or combined among them.

4. A pharmaceutical preparation according to claim 1 for use in the method according to claim 1, **characterized by** containing the capsid protein mixed or combined with antigens capable of inducing a humoral and/or cellular response.

5. A pharmaceutical preparation according to claims 1-4 for use in the method according to claims 1-4, **characterized by** containing the capsid protein mixed with one or several proteins identified in the sequencing list as Sequence ID. NO.: 20, Sequence ID. NO.: 21, Sequence ID. NO.: 22 and Sequence ID. NO.: 23.

6. A pharmaceutical preparation according to claims 1-5 for use in the method according to claim 1 -5, **characterized by** containing the capsid protein fused chemically or genetically to one or several sequences identified in the sequencing list as Sequence ID. NO.: 17, Sequence ID. NO.: 18, Sequence ID. NO.: 19 and Sequence ID. NO.: 24.

7. A pharmaceutical preparation according to claims 1-6 for use in the method according to any one of claims 1-6 **characterized by** containing the capsid protein in aggregated or particulate form.

8. A pharmaceutical preparation according to claims 1-7 for use in the method according to any one of claims 1-7 **characterized by** containing a pharmacologically acceptable vehicle and an oily or not oily adjuvant.

9. A pharmaceutical preparation according to claims 1-8 for use in the method according to any one of claims 1-8 **characterized by** being a preventive or therapeutic agent against the Dengue viruses, for oral, intramuscular, subcutaneous, mucosal or intravenous use.

## Patentansprüche

1. Pharmazeutische Zusammensetzung, **dadurch gekennzeichnet, dass** es sich um einen Impfstoff handelt, der als Immunisierungsantigen das Capsid-Protein von einem oder mehreren Dengue-Virus-Serotypen in rekombinanter Form enthält, zur Verwendung in einem Verfahren zur Induktion einer schützenden Immunreaktion gegen Dengue-Viren in dem empfangenden Organismus durch Immunverstärkung, wobei die Immunverstärkung die verstärkte Induktion antiviraler, serotypspezifischer Antikörper bei einem Patienten durch ein heterologes Dengue-Antigen ist.

2. Pharmazeutische Zusammensetzung nach Anspruch 1 zur Verwendung in dem Verfahren nach Anspruch 1, wobei das heterologe Dengue-Antigen die Domäne III des Hüllproteins eines Dengue-Virus ist.

3. Pharmazeutische Zusammensetzung nach Anspruch 1 zur Verwendung in dem Verfahren nach Anspruch 1, **dadurch gekennzeichnet, dass** sie das Capsid-Protein des Dengue-Virus 1, 2, 3 oder 4 allein, gemischt oder untereinander kombiniert enthält.

4. Pharmazeutische Zusammensetzung nach Anspruch 1 zur Verwendung in dem Verfahren nach Anspruch 1, **dadurch gekennzeichnet, dass** sie das Capsid-Protein enthält, gemischt oder kombiniert mit Antigenen, die in der Lage sind, eine humorale und/oder zelluläre Reaktion zu induzieren.

5. Pharmazeutische Zusammensetzung nach den Ansprüchen 1-4 zur Verwendung in dem Verfahren nach den Ansprüchen 1-4, **dadurch gekennzeichnet, dass** sie das Capsid-Protein enthält, gemischt mit einem oder mehreren Proteinen, identifiziert in der Sequenzliste als Sequenz. ID. NR.: 20, Sequenz ID. NR.: 21, Sequenz ID. NR.: 22 und Sequenz ID. NR.: 23.

6. Pharmazeutische Zusammensetzung nach den Ansprüchen 1-5 zur Verwendung in dem Verfahren nach den Ansprüchen 1-5, **dadurch gekennzeichnet, dass** sie das Capsid-Protein enthält, chemisch oder genetisch verschmolzen mit einer oder mehreren Sequenzen, identifiziert in der Sequenzliste als Sequenz. ID. NR.: 17, Sequenz ID. NR.: 18, Sequenz ID. NR.: 19 und Sequenz ID. NR.: 24.

7. Pharmazeutische Zusammensetzung nach den Ansprüchen 1-6 zur Verwendung in dem Verfahren nach einem der Ansprüche 1-6, **dadurch gekennzeichnet, dass** sie das Capsid-Protein in aggregierter oder partikulärer Form enthält.

8. Pharmazeutische Zusammensetzung nach den Ansprüchen 1-7 zur Verwendung in dem Verfahren nach einem der Ansprüche 1-7, **dadurch gekennzeichnet, dass** sie ein pharmazeutisch akzeptables Vehikel und ein öliges oder nicht öliges Hilfsmittel enthält.

9. Pharmazeutische Zusammensetzung nach den Ansprüchen 1-8 zur Verwendung in dem Verfahren nach einem der Ansprüche 1-8, **dadurch gekennzeichnet, dass** es sich um ein vorbeugendes oder therapeutisches Mittel gegen die Dengue-Viren zur oralen, intramuskulären, subkutanen, mukosalen oder intravenösen Verwendung handelt.

## Revendications

1. Préparation pharmaceutique **caractérisée en ce qu'**elle est un vaccin, ledit vaccin contenant en tant qu'antigène immunisant la protéine de capside d'un ou de plusieurs sérotypes du virus de la dengue sous une forme recombinante, destinée à être utilisée dans un procédé pour induire une réponse immunitaire protectrice contre des virus de la dengue chez l'organisme receveur par immunopotentialisation, dans laquelle ladite immunopotentialisation est l'induction améliorée d'anticorps antiviraux spécifiques à un sérotype chez un sujet par un antigène hétérologue de la dengue.

2. Préparation pharmaceutique selon la revendication 1, destinée à être utilisée dans le procédé selon la revendication 1, dans laquelle ledit antigène hétérologue de la dengue est le domaine III de la protéine d'enveloppe d'un virus de la dengue.

3. Préparation pharmaceutique selon la revendication 1 destinée à être utilisée dans le procédé selon la revendication 1, **caractérisée en ce qu'**elle contient la protéine de capside du virus de la dengue 1, 2, 3 ou 4, seule, mélangée ou combinée entre elles.

4. Préparation pharmaceutique selon la revendication 1 destinée à être utilisée dans le procédé selon la revendication 1, **caractérisée en ce qu'**elle contient la protéine de capside mélangée ou combinée aux antigènes capables d'induire une réponse humorale et/ou cellulaire.

5. Préparation pharmaceutique selon les revendications 1 à 4 destinée à être utilisée dans le procédé selon les revendications 1 à 4, **caractérisée en ce qu'**elle contient la protéine de capside mélangée avec une ou plusieurs protéines identifiées dans la liste des séquences en tant que Sequence ID. N° 20, Sequence ID. N° 21, Sequence ID. N° 22 et Sequence ID. N° 23.

6. Préparation pharmaceutique selon les revendications 1 à 5 destinée à être utilisée dans le procédé selon les revendications 1 à 5, **caractérisée en ce qu'**elle contient la protéine de capside qui est fusionnée chimiquement ou génétiquement à une ou plusieurs séquences identifiées dans la liste des séquences en tant que Sequence ID. N° 17, Sequence ID. N° 18, Sequence ID. N° 19 et Sequence ID. N° 24.

7. Préparation pharmaceutique selon les revendications 1 à 6 destinée à être utilisée dans le procédé selon l'une quelconque des revendications 1 à 6, **caractérisée en ce qu'**elle contient la protéine de capside sous une forme agrégée ou particulaire.

8. Préparation pharmaceutique selon les revendications 1 à 7 destinée à être utilisée dans le procédé selon l'une quelconque des revendications 1 à 7, **caractérisée en ce qu'**elle contient un véhicule pharmacologiquement acceptable et un adjuvant huileux ou non huileux.

9. Préparation pharmaceutique selon les revendications 1 à 8 destinée à être utilisée dans le procédé selon l'une quelconque des revendications 1 à 8, **caractérisée en ce qu'**elle est un agent de prévention ou thérapeutique contre les virus de la dengue, pour une administration par voie orale, intramusculaire, sous-cutanée, muqueuse ou intraveineuse.
